(19)

**Europäisches Patentamt**
**European Patent Office**
**Office européen des brevets**

(11) **EP 4 764 493 A1**

(12) **EUROPEAN PATENT APPLICATION**

(43) Date of publication:
24.06.2026 Bulletin 2026/26

(21) Application number: 24221299.1

(22) Date of filing: 18.12.2024

(51) International Patent Classification (IPC):
*G01N 33/50* (2006.01)   *C12N 5/0775* (2010.01)

(52) Cooperative Patent Classification (CPC):
G01N 33/5044; C12N 5/0663; G01N 33/5023;
G01N 33/5073; G01N 2570/00

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Beiersdorf AG
22529 Hamburg (DE)

(72) Inventors:
• RITTER, Johanna Elisabeth
10439 Berlin (DE)
• SIRACUSA, Annette
22529 Hamburg (DE)
• GRÖNNIGER, Elke
20255 Hamburg (DE)

(74) Representative: Haseltine Lake Kempner LLP
Bürkleinstrasse 10
80538 München (DE)

(54) **REJUVENATING FACTORS**

(57) The present invention relates to a method of identifying rejuvenating factor(s) by culturing bone marrow cells wherein age-specific human serum is added to the bone marrow cells and to a method of testing active agents to augment the effect of the one or more rejuvenating factor(s) identified.

EP 4 764 493 A1

**Description**

FIELD OF THE INVENTION

The present invention relates to a method of identifying rejuvenating factor(s) by culturing bone marrow cells with age-specific serum and to a method of testing active agents of the rejuvenating factor(s) identified. The rejuvenating factor(s) identified and the active agents thereof can be used for treating the signs of aging of skin tissue of a human subject.

BACKGROUND OF THE INVENTION

As the population's life expectancy increases, understanding the mechanisms of aging has gained increased significance. For example, there is an increased interest in the rejuvenation of tissues to promote healthy aging and to reduce the signs of aging. The signs of aging can be reduced, for example, by rejuvenating, augmenting, and/or repairing skin tissue of a human subject.

To date, few approaches are known to rejuvenate tissues, especially in a holistic manner. An example of a known approach is heterochronic parabiosis, wherein the blood circulation systems of two animals of different ages are joined, resulting in rejuvenated tissues within an entire organism (Palovics, R., Keller, A., Schaum, N., Tan, W., Fehlmann, T., Borja, M., Wyss-Coray, T. (2022). Molecular hallmarks of heterochronic parabiosis at single-cell resolution. Nature, 603(7900), 309-314).

However, specific drivers of aging and rejuvenation including the underlying mechanism remain unknown. This is despite the use of tools such as microphysiological systems, also known as organ-on-a-chip platforms, being implemented to recapitulate the human systemic environment.

It is therefore desirable to provide methods to identify key factors of systemic aging, preferably to identify factors for rejuvenating, augmenting, and/or repairing skin tissue of a human subject.

SUMMARY OF THE INVENTION

The present invention is defined in the appended claims.

In accordance with a first aspect, there is provided a method of culturing bone marrow cells in a culture device wherein a growth medium comprising age-specific human serum is added to the bone marrow cells.

In certain embodiments, the age-specific human serum is young human serum from human donors aged 30 years or below.

In certain embodiments, the age-specific human serum is old human serum from human donors aged 60 years or above.

In accordance with a second aspect, there is provided a method of identifying one or more rejuvenating factor(s) comprising

> a) culturing bone marrow cells according to the method according to the first aspect, wherein the growth medium comprises young human serum,
> b) analysing the protein expression of the cultured bone marrow cells of step a),
> c) culturing bone marrow cells according to the method according to the first aspect, wherein the growth medium comprises old human serum,
> d) analysing the protein expression of the cultured bone marrow cells of step c),
> e) determining upregulated protein expression or downregulated protein expression by comparing the protein expression of a pair of bone marrow cell samples according to step b) and step d);
> f) carrying out steps a) to e) for a total of 5 pairs of bone marrow cell samples;
> g) selecting proteins that are regulated in the same direction in at least 4 out of 5 pairs of bone marrow cell samples; and
> h) selecting proteins from step g) that belong to the human secretome.

In accordance with a third aspect, there is provided a method of testing an active agent to augment the effect of the one or more rejuvenating factor(s) identified by the method according to the second aspect, comprising the steps of:

> a) providing skin tissue cells,
> b) determining
>
>> i) the expression level of one or more rejuvenating factor(s) of the skin tissue cells identified by the method according to the second aspect, and/or
ii) hallmarks of aging of the skin tissue cells,

c) contacting the skin cells of step a) with an active agent,
d) determining

i) the expression level of one or more rejuvenating factor(s) of the skin tissue cells identified by the method according to the second aspect, and/or
ii) hallmarks of aging of the skin tissue cells,

e) comparing

i) the expression level of at least one rejuvenating factor determined in step b)i) with the expression level determined in step d)i), and/or
ii) the hallmarks of aging determined in step b)ii) with the hallmarks of aging determined in step d)ii).

In accordance with a fourth aspect, there is provided a composition comprising at least one active agent tested by the method according to the third aspect.

In accordance with a fifth aspect, there is provided the use of a composition according to the fourth aspect for rejuvenating, augmenting, and/or repairing skin tissue of a human subject, wherein the composition is a cosmetic composition. In accordance with a sixth aspect, the composition according to the fourth aspect is provided for use in a method for the treatment of wrinkling, folds, sagging, age spots, uneven pigmentation, thinning, elasticity, scarring, surface roughness, surface vessels, redness, pore size, impaired wound healing, pale complexion, loss of elasticity and/or tightness wherein an effective amount of the composition is administered topically to the skin tissue of the human subject.

Certain embodiments of the present invention may provide one or more of the following advantages:

- the ability to identify rejuvenating factors relating to aging based on novel methods involving culturing bone marrow cells; the culturing method described herein has allowed the identification of specific rejuvenating factors produced by bone marrow cells which was not previously possible;
- identifying rejuvenating factors that have a direct correlation with skin aging which is in contrast to the previous situation, where it was only possible to look at the whole serum, which contained a huge number of factors, without being able to test them all individually and effectively;
- the ability to target the regulation of rejuvenating factors to reduce the signs of aging, such as skin aging;
- an efficient way of testing active agents of one of more of the newly identified rejuvenating factors that have an impact on the appearance of human skin;
- a cost effective way of testing active agents of one of more of the newly identified rejuvenating factors that have an impact on the appearance of human skin;
- a cost-effective way of identifying rejuvenating factors and of testing active agents of one of more of the newly identified rejuvenating factors compared to an in vivo study;
- the ability of directly using rejuvenating factors to reduce signs of aging.

The details, examples and preferences provided in relation to any particular one or more of the stated aspects of the present invention apply equally to all aspects of the present invention. Any combination of the embodiments, examples and preferences described herein in all possible variations thereof is encompassed by the present invention unless otherwise indicated herein, or otherwise clearly contradicted by context.

BRIEF DESCRIPTION OF THE DRAWINGS

The invention will further be illustrated by reference to the following figures:

Fig. 1   Successful co-cultivation of skin models and bone marrow cells in a long-term dynamic in vitro microfluidic organ-on-a chip culture device.

Fig. 1(a)   shows the timeline of the cultivation of skin tissue cells and bone marrow cells. Human bone marrow-MSCs were pre-cultured statically on a hydroxyapatite coated zirconiumoxide based Sponceram® scaffold for 7 days before adding human BM-CD34+ cells and transferring them to the HUMIMIC Chip3 plus. After two weeks, the skin model (Phenion® full-thickness long life insert skin models) were added to the HUMIMIC Chip3plus for another 3 weeks.

Fig. 1(b)   shows the measurement of LDH release in the supernatant of the co-culture. Cytotoxicity was determined as the percentage of released LDH normalized to the maximum LDH release of the skin tissue cells and bone marrow cells after induced lysis.

Fig. 1(c)    shows hematoxylin and eosin (left) and immunofluorescence (right) staining of Collagen IV (Col IV), Keratin 14 (Kr14) and Keratin 10 (Kr10) of the skin tissue cells. Representative images, scale bar = 100 $\mu$m.

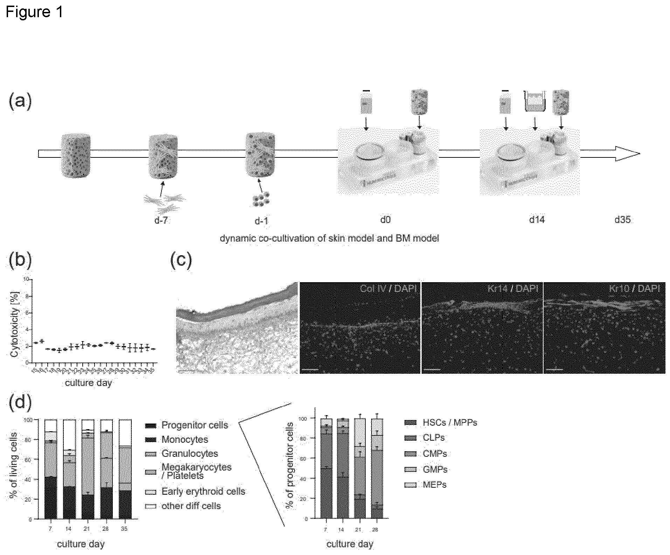

Fig. 1(d)    shows the proportions of different bone marrow cell populations determined using flow cytometry. Left, the percentage of all bone marrow cell populations (progenitor cells, monocytes, granulocytes, platelets/ megakaryocytes, and early erythroids) among living cells is shown. Right, the percentage of progenitor cell populations such as hematopoietic stem cells (HSCs) and multipotent progenitors (MPPs), common lymphoid progenitors (CLPs), common myeloid progenitors (CMPs), granulocyte-monocyte progenitors (GMPs) and megakaryocyte-erythrocyte progenitors (MEPs) among all progenitor cells is depicted. Data are shown as mean values +/- SEM obtained from 1 experiment with 1-2 replicates.

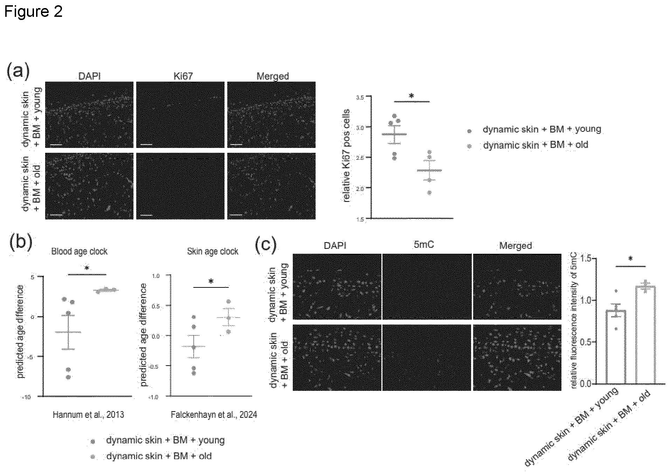

Fig. 2    Young human serum has rejuvenating effects on the skin model in presence of the bone marrow cells in a long-term dynamic *in vitro* microfluidic organ-on-a chip culture device. The bone marrow cells were pre-cultured in the HUMIMIC Chip3plus. After two weeks (culture day 14), the skin model (Phenion® full-thickness long life insert skin models) were added to the system and the co-culture treated with either young human serum or old human serum for three weeks (culture day 14-35). Fig. 2 (a) shows immunofluorescence staining of Ki67 of the skin tissue cells. Representative images (scale bar= 100 $\mu$m) are shown on the left. Bar graphs show the relative proportion of Ki67 positive cells normalized to the control without serum.

Fig. 2(b)    shows the determination of the DNA methylation-based biological age of the skin tissue cells using the skin DNA methylation clock (Falckenhayn, C., Bienkowska, A., Sohle, J., Wegner, K., Raddatz, G., Kristof, B., Gronniger, E. (2023). Identification of dihydromyricetin as a natural DNA methylation inhibitor with rejuvenating activity in human skin. Front Aging, 4, 1258184.) and the blood age clock (Hannum, G., Guinney, J., Zhao, L., Zhang, L., Hughes, G., Sadda, S., Zhang, K. (2013). Genome-wide methylation profiles reveal quantitative views of human aging rates. Mol Cell, 49(2), 359-367).

Fig. 2(c)    shows the immunofluorescence staining of 5-methyl-cytosines of the skin tissue cells. Representative images (scale bar= 100 $\mu$m) are shown on the left. Bar graphs show the relative proportion of 5-methyl-cytosines+ cells normalized to the control without serum. Data were obtained from one experiment with 4-5 replicates, shown as mean values +/- SEM. *p<0.05

Fig. 3    Human serum influences characteristics of the bone marrow cells. Bone marrow cells were precultured in the HUMIMIC Chip3plus. After two weeks (culture day 14), the skin models (Phenion® full-thickness long life insert skin models) were added to the system and the co-culture treated with either young human serum or old human serum for three weeks (culture day 14-35).

Fig. 3(a)    shows the flow cytometric analysis of bone marrow cells. Bar graphs indicate the proportion of progenitor cells (left), monocytes (middle) and granulocytes (right) of all live bone marrow cells after 14, 21, 28 and 35 days of culture.

Fig. 3(b)    shows the mitochondrial membrane potential of bone marrow cells harvested on culture day 17, 24 and 31 normalized to control culture without serum.

Fig. 3(c)    shows the determination of the DNA methylation-based biological age of the bone marrow cells according to the blood age clock (Hannum, G., Guinney, J., Zhao, L., Zhang, L., Hughes, G., Sadda, S., Zhang, K. (2013). Genome-wide methylation profiles reveal quantitative views of human aging rates. Mol Cell, 49(2), 359-367). Data were obtained from two (a) or one (b + c) experiment with 5 replicates, presented as mean values +/- SEM. *p<0.05, **p<0.01.

Fig. 4    The bone marrow cells secrete age-associated proteins. The bone marrow cells were statically cultured for five weeks. After two weeks, the bone marrow cells were treated with either young human serum or old human serum. On culture day 35, the bone marrow cells were harvested, and the cell pellet analysed using tandem LC-MS/MS proteomics.

Fig. 4 (a)    represents the Log2(fold change) and the log2(p value) of up or downregulated rejuvenating factors in the bone marrow cells treated with old human serum compared to young human serum. Rejuvenating factors regulated in the same direction in at least 4 of 5 samples were counted as upregulated or downregulated and are depicted as well.

Fig. 4(b)    shows a comparison of identified rejuvenating factors to the 2772 known human secretome proteins, thereby creating an overlap of 233.

Fig. 4(c)    shows down- (left) and up- (right) regulated gene ontology terms of the overlapped proteins shown in Fig. 4 b).

Fig. 4(d)    shows the overlap of regulated proteins that belong to the human secretome (left) and secretome proteins that significantly change upon aging (right). 55 proteins shared between the two categories are represented in the middle.

Fig. 4(e)     shows the STRING protein network of the down- (left) and up- (right) regulated proteins from the overlap shown in Fig. 4 (d). Expression by different bone marrow cell types is highlighted, i.e. granulocytes, progenitor cells or monocytes. Data were obtained from one experiment with 5 replicates.

Fig. 5     Age-associated downregulated proteins secreted by the bone marrow cells cultured with old human serum compared to bone marrow cells cultured with young human serum rejuvenate skin tissue cells. Old fibroblasts and keratinocytes were statically cultured and treated with 100 ng/ml of the appropriate downregulated age-associated rejuvenating factor for 72 hours.

Fig. 5(a)     shows the immunofluorescence staining of Ki67 of fibroblasts and keratinocytes. Bar graphs show the relative proportion of Ki67+ cells normalized to the control without protein.

Fig. 5(b)     shows a heatmap showing relative gene expression of statically cultured fibroblasts treated with rejuvenating factors normalized to the control cultured without rejuvenating factors.

Fig. 5(c)     shows the relative production of procollagen 1 normalized to the control cultured without rejuvenating factors, measured in the supernatant of treated fibroblasts.

Fig.5 (d)     shows the relative production of hyaluronic acid normalized to the control cultured without rejuvenating factors, measured in the supernatant of treated fibroblasts.

Fig. 5(e)     shows the relative ability of fibroblasts to differentiate into adipocytes of cells treated with rejuvenating factors normalized to the control cultured without rejuvenating factors.

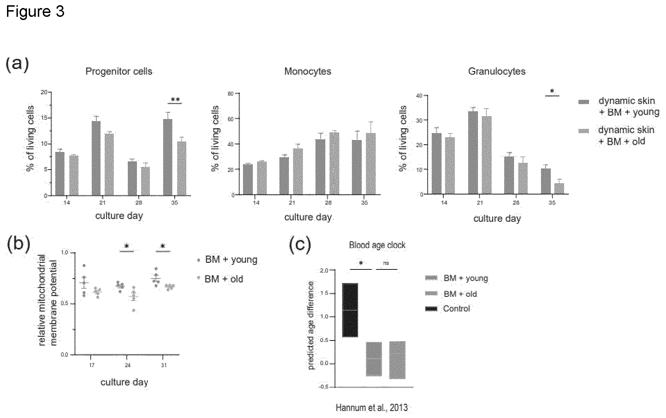

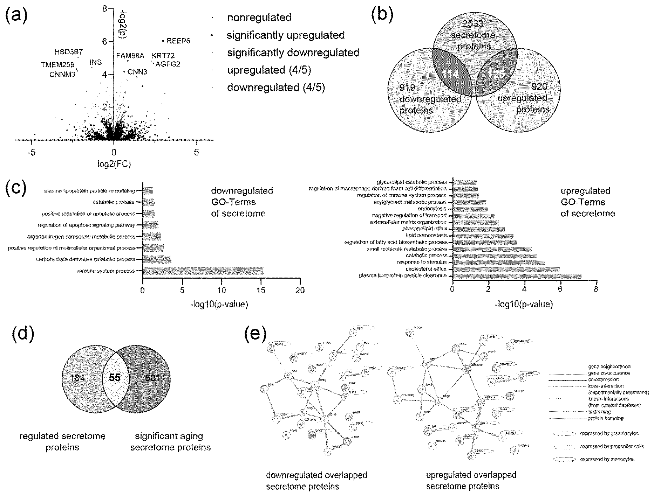

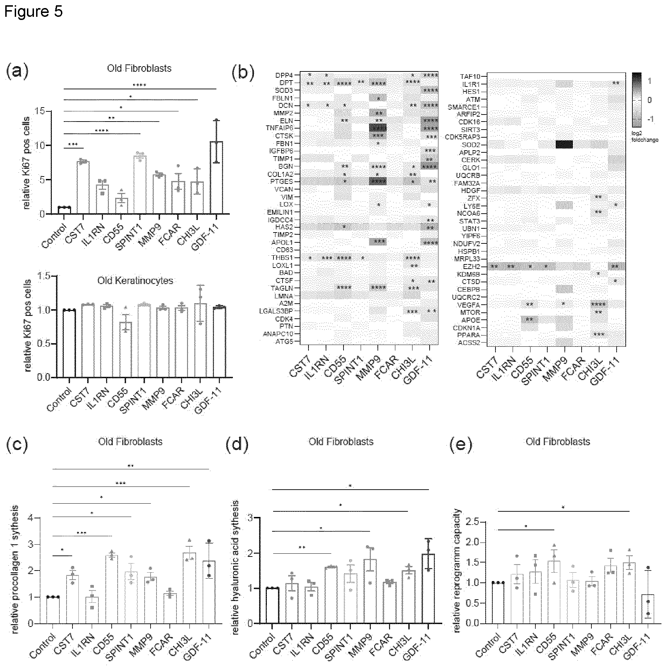

Based on the surprising finding that young human serum has rejuvenating effects on skin tissue cells in presence of bone marrow cells in a long-term dynamic *in vitro* microfluidic organ-on-a chip culture device as shown in Figure 2 and that human serum influences characteristics of the bone marrow cells as shown in Figure 3, the present invention relates to a method of identifying rejuvenating factor(s) by successful cultivation of bone marrow cells in a long-term dynamic *in vitro* microfluidic organ-on-a chip culture device as shown in Figure 1 and comparison of the proteome of bone marrow cells cultured and treated with old and young human serum as exemplified in Figure 4. The present inventors have shown in Figure 5 that age-associated downregulated proteins secreted by the bone marrow cells cultured with young human serum rejuvenate skin tissue cells.

It is understood that the following description and references to the figures concern exemplary embodiments of the present invention and shall not be limiting the scope of the claims.

DETAILED DESCRIPTION

The present invention relates to the surprising finding that heterochronic parabiosis experiments, an approach of joining blood circulation systems of two animals of different ages, can be translated to an *in vitro* cell culture in order to investigate aging. This method has allowed rejuvenation to be studied systemically as well as to elucidate the molecular mechanisms driving age-related changes in the skin. Using this method, it has been possible to identify rejuvenating factors related to the signs of aging. The rejuvenating factors were previously not validated to have a direct effect on aging, especially skin aging. This surprising discovery has opened the path to better understand and treat the signs of aging, which were previously not available.

Identifying the rejuvenating factors according to the method of this present invention has been made possible by the culturing method described herein. The present inventors could show for the first time *in vitro* that young human serum containing several systemic factors rejuvenates the human skin in the presence of bone marrow-derived cells and could attribute the bone marrow cell-dependent rejuvenating effect of systemic factors to bone marrow cell-secreted proteins.

Using the method of culturing bone marrow cells in a microfluidic culture device as described herein, the present inventors were able to reproduce systemic rejuvenating effects of circulating blood factors on the human skin which have been so far only demonstrated in rodent heterochronic parabiosis studies. Moreover, the present inventors identified several proteins as rejuvenating factors to rejuvenate skin tissue cells.

In certain embodiments, bone marrow cells are pre-cultured in a static culture or in a dynamic culture.

In certain embodiments, culturing bone marrow cells comprises co-culturing bone marrow cells with skin tissue cells. Co-culturing bone marrow cells with skin tissue cells refers to a dynamic culture of skin tissue cells and bone marrow cells. Both cell types are cultured together.

A static culture refers to a culture in a vessel such as a plate (for example 6 or 24-well plate) in a growth medium that supplies essential nutrient. A dynamic culture refers to a culture performed in a microfluidic environment such as a microfluidic organ-on-a-chip culture device comprising a growth medium.

In certain embodiments, the culture device is a microfluidic culture device. In certain embodiments, the microfluidic culture device is a microfluidic organ-on-a chip culture device. In certain embodiments, the microfluidic organ-on-a chip culture device is the HUMIMIC Chip3plus (TissUse GmbH) or the GUIDED MODEL Bone-Marrow Chip (Emulate).

In certain embodiments, the age-specific human serum is young human serum from human donors aged 30 years or below. In certain embodiments, the age-specific human serum is old human serum from human donors aged 60 years or above. In certain embodiments, the young human serum is a mix of sera from 2 to 20 donors aged 30 years or below. In certain embodiments, the old human serum is a mix of sera from 2 to 20 donors aged 60 years or above. In certain embodiments, the young human serum is a mix of sera of at least 5 donors aged 30 years or below. In certain embodiments, the old human serum is a mix of sera of at least 5 donors aged 60 years or above.

In certain embodiments, the growth medium is any cell culture medium or substrate that supplies essential nutrients. In certain embodiments, the growth medium is StemSpan™ SFEM II (Stemcell Technologies). In certain embodiments, the growth medium is StemSpan™ SFEM II adding 1-250 ng/mL stem cell factor, 1-250 ng/mL thyroid peroxidase, 1-250 ng/mL Fms-related tyrosine kinase 3 ligand (Flt3-L), and 0,1-2 % Penicillin/Streptomycin. In certain embodiments, the growth medium is StemSpan™ SFEM II adding 50 ng/mL stem cell factor, 10 ng/mL thyroid peroxidase, 100 ng/mL Fms-related tyrosine kinase 3 ligand (Flt3-L), and 1 % Penicillin/Streptomycin.

In certain embodiments, the bone marrow cells or the bone marrow cells co-cultured with skin tissue cells are cultivated in a growth medium comprising about 1 vol.% to about 20 vol.% of age-specific human serum, such as about 3 vol.% to about 17 vol.%, about 5 vol.% to about 15 vol.%, about 8 vol.% to about 12 vol.%, based on the total vol.% of the serum. For example, the growth medium comprises about 1 vol.%, about 2 vol.%, about 3 vol.%, about 4 vol.%, about 5 vol.%, about 6 vol.%, about 7 vol.%, about 8 vol.%, about 9 vol.%, about 10 vol.%, about 11 vol.%, about 12 vol.%, about 13 vol.%, about 14vol.%, about 15vol.%, about 16 vol.%, about 17 vol.%, about 18 vol.%, about 19 vol.% or about 20 vol.% of age-specific human serum based on the total vol.% of the serum. In certain embodiments, the growth medium comprises about 10 vol.% age-specific human serum based on the total vol.% of the serum.

In certain embodiments, the bone marrow cells are pre-cultured for a period of time of between about 1 day to about 21 days. For example, the bone marrow cells are pre-cultured for a period of time of between about 5 days to about 20 days, for a period of time of between about 10 days to about 20 days, or for a period of time of between about 15 days to about 20 days, for a period of time of between about 5 days to about 15 days, for a period of time of between about 10 days to about 15 days, or for a period of time of between about 12 days to about 15 days. For example, the bone marrow cells are pre-cultured for about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, or about 21 days. In certain embodiments, the bone marrow cells are pre-cultivated for a period of time of about 21 days. In a preferred embodiment, the bone marrow cells are statically pre-cultured for 7 days, followed by dynamic pre-culturing for about 14 days.

In certain embodiments, culturing bone marrow cells comprises co-culturing bone marrow cells with skin tissue cells. Therefore, skin tissue cells are added to the bone marrow cells after a period of time of pre-culturing bone marrow cells of about 10 days to about 21 days. For example, after a period of time of pre-culturing bone marrow cells of about 10 days, about 11 days, about 12 days, about 13 days, about 14 days, about 15 days, about 16 days, about 17 days, about 18 days, about 19 days, about 20 days or about 21 days. In a preferred embodiment, skin tissue cells are added to the bone marrow cells after a period of time of pre-culturing bone marrow cells of about 14 days.

In certain embodiments, the bone marrow cells and the skin tissue cells are co-cultured for a period of time of between about 1 day to about 35 days. For example, the bone marrow cells and the skin tissue cells are co-cultured for a period of time of between about 5 days to about 30 days, for a period of time of between about 10 days to about 30 days, or for a period of time of between about 15 days to about 30 days, for a period of time of between about 5 days to about 25 days, for a period of time of between about 10 days to about 20 days, or for a period of time of between about 20 days to about 30 days. For example, the bone marrow cells and the skin tissue cells are co-cultured for about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, about 30 days, about 31 days, about 32 days, about 33 days, about 34 days or about 35 days. In a preferred embodiment, the bone marrow cells and the skin tissue cells are co-cultivated for a period of time of 21 days.

In certain embodiments, the bone marrow cells and the skin tissue cells are cultured comprising the steps of pre-culturing bone marrow cells and co-culturing bone marrow cells and skin tissue cells for a period of time of between about 1 day to about 42 days. For example, the bone marrow cells and the skin tissue cells are cultured for a period of time of between about 5 days to about 30 days, for a period of time of between about 10 days to about 30 days, or for a period of time of between about 15 days to about 30 days, for a period of time of between about 5 days to about 35 days, for a period of time of between about 10 days to about 35 days, or for a period of time of between about 25 days to about 35 days. For example, the bone marrow cells and the skin tissue cells are cultured for about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, about 30 days, about 31 days, about 32 days, about 33 days, about 34 days, about 35 days, about 36 days, about 37 days, about 38 days, about 39 days, about 40 days, about 41 days or about 42 days.

In certain embodiments, age-specific human serum is added to the growth medium for a period of time from about culture day 14 to about culture day 42. The term "culture day" refers to the day of the current cultivation period, calculated from the beginning of the pre-culture period. For example, age-specific human serum is added to the growth medium for a period of time from about culture day 14 to about culture day 21, from about culture day 14 to about culture day 28, from about culture day 14 to about culture day 35 or from about culture day 21 to about culture day 42.

In certain embodiments, the skin tissue cells are human skin tissue cells. Human skin tissue cells used according to the methods of the invention are obtained by harvesting the entire skin tissue cells required from a human individual. Harvesting skin tissue cells from the human individual may be carried out using suction blistering, punch biopsy, shave biopsy or during any surgical procedure such as plastic surgery, lifting, grafting, or the like. The skin tissue cells may be taken from the epidermis or dermis. Human skin tissue cells may be cultured from a small sample of skin tissue cells harvested from a human individual. The harvested human skin tissue cells are grown *in vitro* in a vessel such as a petri dish or a plate in a growth medium that supplies essential nutrient.

In certain embodiments, the human skin tissue cells used according to the method of the invention are obtained by culturing the skin tissue cells using an *in vitro* method. The human skin tissue cells used may be a mixture of harvested cells and cultured cells. In certain embodiments, the skin tissue cells are Phenion® full-thickness insert skin models.

In certain embodiments, the bone marrow cells are human bone marrow cells. Human bone marrow cells used according to the methods of the invention are obtained by harvesting the entire bone marrow cells required from a human individual. In certain embodiments, the bone marrow cells used according to the method of the invention are obtained by culturing the bone marrow cells using an *in vitro* method. In certain embodiments, the bone marrow cells are hBM-MSCs (Lonza Group AG). In certain embodiments, the bone marrow cells are hBM-CD34+ cells (Lonza Group AG). In certain embodiments, the bone marrow cells are a combination of hBM-MSCs and hBM-CD34+ cells.

The bone marrow cells used to identify the one or more rejuvenating factors(s) may be pre-cultured in a static culture or in a dynamic culture. In certain embodiments, bone marrow cells are cultured with age-specific human serum, either with young human serum or with old human serum, followed by subsequently analysing the protein expression of the cultured bone marrow cells. In certain embodiments, bone marrow cells are cultured without age-specific human serum followed by subsequently analysing the protein expression of the cultured bone marrow cells.

In certain embodiments, bone marrow cells and skin tissue cells are co-cultured with age-specific human serum, either with young human serum or with old human serum, followed by subsequently analysing the protein expression of the cultured bone marrow cells. In certain embodiments, bone marrow cells and skin tissue cells are co-cultured without age-specific human serum followed by subsequently analysing the protein expression of the cultured bone marrow cells.

In certain embodiments, analysing the protein expression of the bone marrow cells cultured with age-specific human serum, either with young human serum or with old human serum, is performed by LC-MS/MS proteomics. In certain embodiments, analysing the protein expression of the bone marrow cells cultured without age-specific human serum is performed by LC-MS/MS proteomics.

In one example, the protein expression of bone marrow cells cultured with young human serum (A) was compared with the protein expression of bone marrow cells cultured with old human serum (B). For a single comparison, it was determined whether the expression of a protein was upregulated or downregulated for the bone marrow cells cultured with young human serum (A) versus the bone marrow cells cultured with old human serum (B). This comparison was carried out for 5 pairs of bone marrow cell samples (A and B). It was then determined whether the protein was regulated in the same direction (i.e., upregulated or downregulated) for at least 4 out of 5 pairs of bone marrow cell samples. The proteins identified by this comparison that belong to the human secretome (according to human protein atlas) where then selected.

Therefore, starting from a plethora of proteins, the present inventors identified proteins that were regulated differently in at least 4 out of 5 bone marrow cell samples, either upregulated or downregulated, using the method above. The proteins identified were then narrowed down further by their ability to be potentially secreted to act on other tissues i.e. proteins which belong to the human secretome. More specifically, the proteins identified were compared to proteins known to belong to the human secretome by comparison with the Human Protein Atlas (Human Protein Atlas, list "The human secretome", www.proteinatlas.org and www.proteinatlas.org/humanproteome/tissue/secretome (Uhlén, M., Karlsson, M. J., Hober, A., Svensson, A.-S., Scheffel, J., Kotol, D., Sivertsson, Å. (2019). The human secretome. Science Signaling, 12(609), eaaz0274). Using this method, the following proteins were identified: PLAU, SDCBP, SULF2, APOB, APOC1, APOC3, APOC2, MINPP, PLOD2, PON3, CES2, LIPA, PON2, FKBP10, PLA2G15, TGM2, ARSB, CES1, GLS, GPI, NAAA, ACP3, P4HA1, ALG1, CARM1, NGLY1, NME3, NT5E, P4HA2, PROZ, RDH11, TFRC, IL4I1, NDUFB11, FABP5, GLIPR1, ATP5PB, SDHD, TXN, FAM3C, AGT, YBX1, CEACAM1, ADGRE3, APOC4, APOF, APOM, APOE, S100A13, GBP1, PDCD1LG2, CDC23, COL22A1, COL6A1, COL6A2, COL6A3, CFHR1, C8G, C4A, SEMA3C, THEM6, ADAM9, NCEH1, P3H1, MMP7, PLBD2, CASP7, DHX8, ERAP1, LNPEP, PIK3AP1, DNASE1, IDE, FAP, TEX264, LGALS7, LGALS9, GOLM1, HDGF, CKLF, TGFBI, MYDGF, SHBG, IGKV6D-21, IGLV2-11, ITFG1, HINT2, PF4, MRPL2, MRPL24, SERPINH1, SERPINE1, HSPA1A, DNAJB11, YBX1, CD9, ERLIN2, CRYAB, DAG1, ERLEC1, GNAS, MAGT1, OS9, PDZD8, EMC10, MIA2, POGLUT3, CRP, SAA4, PRXL2A, RTF1, RCN3, BPIFB1, OOSP3, BLTP2, FCN1, HNRNPA2B1, RCN1, SDF2L1, SDF4, LAMC1, PVR, LILRB1, CD14, NRP1, ALCAM, IRAK3, SPINT1, ERO1A, CHIT1, LPL, AUH, CMPK1, FKBP7, ST3GAL2, QPCT, USP11, ACE, CTSA, CTSH, GALC, GLA, HEXA, HMGCL, PLA2G7, PTGS1, GUSB, LCAT, VNN1, OXCT1, CHI3L1, GPX3, ANGPTL4, SLC8A1, MTMR14, LAT2, IL1RN, S100A8, CD55, CD40, CD163, CSF2RA, FAS, FCAR, ITGA4, CCN1, EDEM2, EDEM3, EMILIN1, MFGE8, SEMG1, PCOLCE2, PCYOX1L, KDM1A, MYG1, DPP7, SLPI, CPA4, DDX17, MSRB2, PTPRO, MMP9, DNASE1L1, KDM3B, SCPEP1, ATPAF1, D2HGDH, FGG, CXCL5, IGHG1, IGHV2-5, IGHV3-15, IGHV3-20, IGHV3-35, IGHV4-28, IGKJ1, IGKV1-16, IGKV1-39, IGKV3-11, IGKV3D-15, IGKV6-21, IGLV3-21, IGLV4-69, IGLV7-46, IL18, PRG3, INS, PGLYRP2, SERPINB2, CNPY3, LBP, CETP, INTS3, MATR3, SAA1, MTX2, SYAP1, A2ML1, CST7, CIAO2A, PLBD1, SELENOF, SELENON, COLEC11, INHBA, SIGLEC7, HLA-C, ZG16B, CHID1, MESD, SIL1, TBL2, DMBT1, and variants thereof. Table 1 shows the list of these rejuvenating factors identified, which are proteins found in NCBI-Genes (last release date 10/05/2024) with the following Identifier (ENTREZ ID) (last updated 10/12/2024):

Table 1:

| No. | Protein | ENTREZ ID | No. | Protein | ENTREZ ID |
|-----|---------|-----------|-----|---------|-----------|
| 1 | PVR | 5817 | 118 | APOC3 | 345 |
| 2 | LILRB1 | 10859 | 119 | APOC2 | 344 |
| 3 | CD14 | 929 | 120 | MINPP1 | 9562 |
| 4 | NRP1 | 8829 | 121 | PLOD2 | 5352 |
| 5 | ALCAM | 214 | 122 | PON3 | 5446 |
| 6 | IRAK3 | 11213 | 123 | CES2 | 8824 |

| | | | | | |
|---|---|---|---|---|---|
| 7 | SPINT1 | 6692 | 124 | LIPA | 3988 |
| 8 | ERO1A | 30001 | 125 | PON2 | 5445 |
| 9 | CHIT1 | 1118 | 126 | FKBP10 | 60681 |
| 10 | LPL | 4023 | 127 | PLA2G15 | 23659 |
| 11 | AUH | 549 | 128 | TGM2 | 7052 |
| 12 | CMPK1 | 51727 | 129 | ARSB | 411 |
| 13 | FKBP7 | 51661 | 130 | CES1 | 1066 |
| 14 | ST3GAL2 | 6483 | 131 | GLS | 2744 |
| 15 | QPCT | 25797 | 132 | GPI | 2821 |
| 16 | USP11 | 8237 | 133 | NAAA | 27163 |
| 17 | ACE | 1636 | 134 | ACP3 | 55 |
| 18 | CTSA | 5476 | 135 | P4HA1 | 5033 |
| 19 | CTSH | 1512 | 136 | ALG1 | 56052 |
| 20 | GALC | 2581 | 137 | CARM1 | 10498 |
| 21 | GLA | 2717 | 138 | NGLY1 | 55768 |
| 22 | HEXA | 3073 | 139 | NME3 | 4832 |
| 23 | HMGCL | 3155 | 140 | NT5E | 4907 |
| 24 | PLA2G7 | 7941 | 141 | P4HA2 | 8974 |
| 25 | PTGS1 | 5742 | 142 | PROZ | 8858 |
| 26 | GUSB | 2990 | 143 | RDH11 | 51109 |
| 27 | LCAT | 3931 | 144 | TFRC | 7037 |
| 28 | VNN1 | 8876 | 145 | IL4I1 | 259307 |
| 29 | OXCT1 | 5019 | 146 | NDUFB11 | 54539 |
| 30 | CHI3L1 | 1116 | 147 | FABP5 | 2171 |
| 31 | GPX3 | 2878 | 148 | GLIPR1 | 11010 |
| 32 | ANGPTL4 | 51129 | 149 | ATP5PB | 515 |
| 33 | SLC8A1 | 6546 | 150 | SDHD | 6392 |
| 34 | MTMR14 | 64419 | 151 | TXN | 7295 |
| 35 | LAT2 | 7462 | 152 | FAM3C | 10447 |
| 36 | IL1RN | 3557 | 153 | AGT | 183 |
| 37 | S100A8 | 6279 | 154 | YBX1 | 4904 |
| 38 | CD55 | 1604 | 155 | CEACAM1 | 634 |
| 39 | CD40 | 958 | 156 | ADGRE3 | 84658 |
| 40 | CD163 | 9332 | 157 | APOC4 | 346 |
| 41 | CSF2RA | 1438 | 158 | APOF | 319 |
| 42 | FAS | 355 | 159 | APOM | 55937 |

| | | | | | | |
|---|---|---|---|---|---|---|
| 43 | FCAR | 2204 | 160 | APOE | 348 |
| 44 | ITGA4 | 3676 | 161 | S100A13 | 6284 |
| 45 | CCN1 | 3491 | 162 | GBP1 | 2633 |
| 46 | EDEM2 | 55741 | 163 | PDCD1LG2 | 80380 |
| 47 | EDEM3 | 80267 | 164 | CDC23 | 8697 |
| 48 | EMILIN1 | 11117 | 165 | COL22A1 | 169044 |
| 49 | MFGE8 | 4240 | 166 | COL6A1 | 1291 |
| 50 | SEMG1 | 6406 | 167 | COL6A2 | 1292 |
| 51 | PCOLCE2 | 26577 | 168 | COL6A3 | 1293 |
| 52 | PCYOX1L | 78991 | 169 | CFHR1 | 3078 |
| 53 | KDM1A | 23028 | 170 | C8G | 733 |
| 54 | MYG1 | 60314 | 171 | C4A | 720 |
| 55 | DPP7 | 29952 | 172 | SEMA3C | 10512 |
| 56 | SLPI | 6590 | 173 | THEM6 | 51337 |
| 57 | CPA4 | 51200 | 174 | ADAM9 | 8754 |
| 58 | DDX17 | 10521 | 175 | NCEH1 | 57552 |
| 59 | MSRB2 | 22921 | 176 | P3H1 | 64175 |
| 60 | PTPRO | 5800 | 177 | MMP7 | 4316 |
| 61 | MMP9 | 4318 | 178 | PLBD2 | 196463 |
| 62 | DNASE1L1 | 1774 | 179 | CASP7 | 840 |
| 63 | KDM3B | 51780 | 180 | DHX8 | 1659 |
| 64 | SCPEP1 | 59342 | 181 | ERAP1 | 51752 |
| 65 | ATPAF1 | 64756 | 182 | LNPEP | 4012 |
| 66 | D2HGDH | 728294 | 183 | PIK3AP1 | 118788 |
| 67 | FGG | 2266 | 184 | DNASE1 | 1773 |
| 68 | CXCL5 | 6374 | 185 | IDE | 3416 |
| 69 | IGHG1 | 3500 | 186 | FAP | 2191 |
| 70 | IGHV2-5 | 28457 | 187 | TEX264 | 51368 |
| 71 | IGHV3-15 | 28448 | 188 | LGALS7 | 3963 |
| 72 | IGHV3-20 | 28445 | 189 | LGALS9 | 3965 |
| 73 | IGHV3-35 | 28432 | 190 | GOLM1 | 51280 |
| 74 | IGHV4-28 | 28400 | 191 | HDGF | 3068 |
| 75 | IGKJ1 | 28950 | 192 | CKLF | 51192 |
| 76 | IGKV1-16 | 28938 | 193 | TGFBI | 7045 |
| 77 | IGKV1-39 | 28930 | 194 | MYDGF | 56005 |
| 78 | IGKV3-11 | 28914 | 195 | SHBG | 6462 |

| 79 | IGKV3D-15 | 28875 |
| 80 | IGKV6-21 | 28906 |
| 81 | IGLV3-21 | 28796 |
| 82 | IGLV4-69 | 28784 |
| 83 | IGLV7-46 | 28775 |
| 84 | IL18 | 3606 |
| 85 | PRG3 | 10394 |
| 86 | INS | 3630 |
| 87 | PGLYRP2 | 114770 |
| 88 | SERPINB2 | 5055 |
| 89 | CNPY3 | 10695 |
| 90 | LBP | 3929 |
| 91 | CETP | 1071 |
| 92 | INTS3 | 65123 |
| 93 | MATR3 | 9782 |
| 94 | SAA1 | 6288 |
| 95 | MTX2 | 10651 |
| 96 | SYAP1 | 94056 |
| 97 | A2ML1 | 144568 |
| 98 | CST7 | 8530 |
| 99 | CIAO2A | 84191 |
| 100 | PLBD1 | 79887 |
| 101 | SELENOF | 9403 |
| 102 | SELENON | 57190 |
| 103 | COLEC11 | 78989 |
| 104 | INHBA | 3624 |
| 105 | SIGLEC7 | 27036 |
| 106 | HLA-C | 3107 |
| 107 | ZG16B | 124220 |
| 108 | CHID1 | 66005 |
| 109 | MESD | 23184 |
| 110 | SIL1 | 64374 |
| 111 | TBL2 | 26608 |
| 112 | DMBT1 | 1755 |
| 113 | PLAU | 5328 |
| 114 | SDCBP | 6386 |

| 196 | IGKV6D-21 | 28870 |
| 197 | IGLV2-11 | 28816 |
| 198 | ITFG1 | 81533 |
| 199 | HINT2 | 84681 |
| 200 | PF4 | 5196 |
| 201 | MRPL2 | 51069 |
| 202 | MRPL24 | 79590 |
| 203 | SERPINH1 | 871 |
| 204 | SERPINE1 | 5054 |
| 205 | HSPA1A | 3303 |
| 206 | DNAJB11 | 51726 |
| 207 | YBX1 | 4904 |
| 208 | CD9 | 928 |
| 209 | ERLIN2 | 11160 |
| 210 | CRYAB | 1410 |
| 211 | DAG1 | 1605 |
| 212 | ERLEC1 | 27248 |
| 213 | GNAS | 2778 |
| 214 | MAGT1 | 84061 |
| 215 | OS9 | 10956 |
| 216 | PDZD8 | 118987 |
| 217 | EMC10 | 284361 |
| 218 | MIA2 | 4253 |
| 219 | POGLUT3 | 143888 |
| 220 | CRP | 1401 |
| 221 | SAA4 | 6291 |
| 222 | PRXL2A | 84293 |
| 223 | RTF1 | 23168 |
| 224 | RCN3 | 57333 |
| 225 | BPIFB1 | 92747 |
| 226 | OOSP3 | 112577461 |
| 227 | BLTP2 | 9703 |
| 228 | FCN1 | 2219 |
| 229 | HNRNPA2B1 | 3181 |
| 230 | RCN1 | 5954 |
| 231 | SDF2L1 | 23753 |

| 115 | SULF2 | 55959 |
|-----|-------|-------|
| 116 | APOB | 338 |
| 117 | APOC1 | 341 |

| 232 | SDF4 | 51150 |
|-----|------|-------|
| 233 | LAMC1 | 3915 |

In an additional step, the present inventors compared the proteins identified in Table 1 with proteins that have generally been reported to change upon aging inside the human plasma. However the effect of the proteins identified has been investigated reported or investigated further. (Coenen, L., Lehallier, B., de Vries, H. E., & Middeldorp, J. (2023). Markers of aging: Unsupervised integrated analyses of the human plasma proteome. Front Aging, 4, 1112109; Lehallier, B., Gate, D., Schaum, N., Nanasi, T., Lee, S. E., Yousef, H., Wyss-Coray, T. (2019). Undulating changes in human plasma proteome profiles across the lifespan. Nat Med, 25(12), 1843-1850.; Moaddel, R., Ubaida-Mohien, C., Tanaka, T., Lyashkov, A., Basisty, N., Schilling, B., Ferrucci, L. (2021). Proteomics in aging research: A roadmap to clinical, translational, research. Aging Cell, 20(4), e13325). Using this analysis, the present inventors found a further group of proteins that were regulated differently in bone marrow cells cultured with the addition of young human serum compared with the addition of old human serum, and which also have been previously described to be differentially expressed in aged human serum versus young human serum. In this way, the present inventors were thus able to identify a sub-group of rejuvenating factor(s) out of many thousands of proteins. The sub-group of rejuvenating factors identified by the present inventors are as follows: APOF, SAA4, PLOD2, TXN, ARSB, HNRNPA2B1, LGALS7, NAAA, SULF2, GOLM1, ERLEC1, SDF2L1, NDUFB11, DNAJB11, MINPP1, LGALS9, PLAU, APOB, SERPINE1, MMP7, TGFBI, S100A13, ERAP1, HSPA1A, CRP, GPI, CEACAM1, PCYOX1L, ALCAM, CD163, CTSA, QPCT, CHIT1, SPINT1, CST7, FGG, SLPI, CD55, INHBA, MMP9, IL1RN, FAS, CHI3L1, MFGE8, CPA4, PRG3, CTSH, SAA1, FCAR/CD89, PLBD1, LILRB1, SIGLEC7, and FKBP7.

As used herein, the term "rejuvenating factor(s)" refers to circulating blood factor(s) and/or proteins secreted by the bone marrow cells, which are identified according to a method of the invention to have a rejuvenating effect. In certain embodiments, the rejuvenating factor is a plasma protein secreted by the bone marrow cells, which are identified according to a method of the invention to have a rejuvenating effect. In certain embodiments, the rejuvenating factor(s) belong(s) to the human secretome according to the human protein atlas (Human Protein Atlas, list "The human secretome", www.proteinatlas.org and www.proteinatlas.org/humanproteome/tissue/secretome).

In certain embodiments, the rejuvenating factor is an upregulated systemic protein or a downregulated systemic protein with aging.

In certain embodiments, the rejuvenating factor is a upregulated systemic protein with aging selected from the group comprising PLAU, SDCBP, SULF2, APOB, APOC1, APOC3, APOC2, MINPP, PLOD2, PON3, CES2, LIPA, PON2, FKBP10, PLA2G15, TGM2, ARSB, CES1, GLS, GPI, NAAA, ACP3, P4HA1, ALG1, CARM1, NGLY1, NME3, NT5E, P4HA2, PROZ, RDH11, TFRC, IL4I1, NDUFB11, FABP5, GLIPR1, ATP5PB, SDHD, TXN, FAM3C, AGT, YBX1, CEACAM1, ADGRE3, APOC4, APOF, APOM, APOE, S100A13, GBP1, PDCD1LG2, CDC23, COL22A1, COL6A1, COL6A2, COL6A3, CFHR1, C8G, C4A, SEMA3C, THEM6, ADAM9, NCEH1, P3H1, MMP7, PLBD2, CASP7, DHX8, ERAP1, LNPEP, PIK3AP1, DNASE1, IDE, FAP, TEX264, LGALS7, LGALS9, GOLM1, HDGF, CKLF, TGFBI, MYDGF, SHBG, IGKV6D-21, IGLV2-11, ITFG1, HINT2, PF4, MRPL2, MRPL24, SERPINH1, SERPINE1, HSPA1A, DNAJB11, YBX1, CD9, ERLIN2, CRYAB, DAG1, ERLEC1, GNAS, MAGT1, OS9, PDZD8, EMC10, MIA2, POGLUT3, CRP, SAA4, PRXL2A, RTF1, RCN3, BPIFB1, OOSP3, BLTP2, FCN1, HNRNPA2B1, RCN1, SDF2L1, SDF4, LAMC1, and variants thereof.

In certain embodiments, the rejuvenating factor is a downregulated systemic protein with aging selected from the group comprising PVR, LILRB1, CD14, NRP1, ALCAM, IRAK3, SPINT1, ERO1A, CHIT1, LPL, AUH, CMPK1, FKBP7, ST3GAL2, QPCT, USP11, ACE, CTSA, CTSH, GALC, GLA, HEXA, HMGCL, PLA2G7, PTGS1, GUSB, LCAT, VNN1, OXCT1, CHI3L1, GPX3, ANGPTL4, SLC8A1, MTMR14, LAT2, IL1RN, S100A8, CD55, CD40, CD163, CSF2RA, FAS, FCAR, ITGA4, CCN1, EDEM2, EDEM3, EMILIN1, MFGE8, SEMG1, PCOLCE2, PCYOX1L, KDM1A, MYG1, DPP7, SLPI, CPA4, DDX17, MSRB2, PTPRO, MMP9, DNASE1L1, KDM3B, SCPEP1, ATPAF1, D2HGDH, FGG, CXCL5, IGHG1, IGHV2-5, IGHV3-15, IGHV3-20, IGHV3-35, IGHV4-28, IGKJ1, IGKV1-16, IGKV1-39, IGKV3-11, IGKV3D-15, IGKV6-21, IGLV3-21, IGLV4-69, IGLV7-46, IL18, PRG3, INS, PGLYRP2, SERPINB2, CNPY3, LBP, CETP, INTS3, MATR3, SAA1, MTX2, SYAP1, A2ML1, CST7, CIAO2A, PLBD1, SELENOF, SELENON, COLEC11, INHBA, SIGLEC7, HLA-C, ZG16B, CHID1, MESD, SIL1, TBL2, DMBT1, and variants thereof.

In certain embodiments, the rejuvenating factor is a protein expressed by monocytes selected from the group comprising IRAK3, GUSB, LCAT, VNN1, MTMR14, FAS, FCAR, ITGA4, DNASE1L1, KDM3B, SCPEP1, INS, HLA-C, ZG16B, SDCBP, SULF2, ARSB, CES1, GLS, GPI, NAAA, ACP3, P4HA1, TFRC, NDUFB11, SDHD, TXN, YBX1, ADGRE3, GBP1, CASP7, DHX8, ERAP1, LNPEP, PIK3AP1, LGALS9, GOLM1, TGFBI, HSPA1A, DNAJB11, YBX1, DAG1, ERLEC1, GNAS, MAGT1, OS9, PDZD8, BLTP2, FCN1, HNRNPA2B1, and variants thereof.

In certain embodiments, the rejuvenating factor is a protein expressed by granulocytes selected from the group comprising CHIT1, QPCT, USP11, IL1RN, CD55, MFGE8, DPP7, SLPI, CPA4, IL18, MTX2, SYAP1, A2ML1, CST7, S100A13, PDCD1LG2, CKLF, CD9, PRXL2A, RTF1, and variants thereof.

In certain embodiments, the rejuvenating factor is a protein expressed by macrophages selected from the group comprising PVR, LILRB1, CD14, NRP1, SPINT1, LPL, ACE, CTSA, CTSH, GALC, GLA, HEXA, HMGCL, PLA2G7, PTGS1, CHI3L1, GPX3, ANGPTL4, SLC8A1, LAT2, S100A8, CD40, CD163, CSF2RA, CCN1, SEMG1, PCOLCE2, DDX17, MSRB2, PTPRO, MMP9 C, XCL5, PGLYRP2, SERPINB2, CNPY3, CETP, CIAO2A, PLBD1, SELENOF, SELENON, COLEC11, INHBA, SIGLEC7, PLAU, APOC2, CES2, LIPA, PON2, FKBP10, PLA2G15, TGM2, FABP5, GLIPR1, CEACAM1, APOE, COL22A1, COL6A1, COL6A2, COL6A3, C8G, SEMA3C, ADAM9, NCEH1, P3H1, MMP7, PLBD2, TEX264, LGALS7, SHBG, SERPINH1, SERPINE1, ERLIN2, CRYAB, RCN3, BPIFB1, OOSP3, and variants thereof.

In certain embodiments, the rejuvenating factor is a protein expressed by plasma cells selected from the group comprising ALCAM, ERO1A, OXCT1, EDEM2, EDEM3, ATPAF1, D2HGDH, IGHG1, IGHV2-5, IGHV3-15, IGHV3-20, IGHV3-35, IGHV4-28, IGKJ1, IGKV1-16, IGKV1-39, IGKV3-11, IGKV3D-15, IGKV6-21, IGLV3-21, IGLV4-69, IGLV7-46, CHID1, MESD, SIL1, TBL2, ALG1, CARM1, NGLY1, NME3, NT5E, P4HA2, PROZ, RDH11, FAM3C, AGT, C4A, DNASE1, IDE, FAP, MYDGF, IGKV6D-21, IGLV2-11, ITFG1, HINT2, MRPL24, EMC10, MIA2, RCN1, SDF2L1, SDF4, LAMC1, and variants thereof.

In certain embodiments, the rejuvenating factor is a protein expressed by erythroid cells selected from the group comprising AUH, CMPK1, FKBP7, ST3GAL2, EMILIN1, KDM1A, MYG1, FGG, PRG3, LBP, INTS3, MATR3, SAA1, APOB, APOC1, APOC3, MINPP1, PLOD2, IL4I1, APOC4, APOF, APOM, CDC23, CFHR1, THEM6, HDGF, MRPL2, POGLUT3, CRP, SAA4, and variants thereof.

In certain embodiments, the rejuvenating factor is a protein expressed by Kupffer cells such as PON3, and variants thereof.

In certain embodiments, the rejuvenating factor is a protein expressed by progenitor cells selected from the group comprising of PVR, SPINT1, ERO1A, AUH, CMPK1, FKBP7, ST3GAL2, QPCT, USP11, ACE, CTSA, CTSH, GALC, GLA, HEXA, HMGCL, GUSB, LCAT, OXCT1, MTMR14, CD55, CD40, FAS, CCN1, EDEM2, EDEM3, MFGE8, PCYOX1L, KDM1A, MYG1, DPP7, SLPI, DDX17, MSRB2, PTPRO, DNASE1L1, KDM3B, SCPEP1, ATPAF1, D2HGDH, IL18, PRG3, CNPY3, INTS3, MATR3, MTX2, SYAP1, CIAO2A, PLBD1, SELENOF, SELENON, HLA-C, ZG16B, CHID1, MESD, SIL1, TBL2, DMBT1, SDCBP, SULF2, MINPP1, PLOD2, CES2, LIPA, PON2, ARSB, CES1, GLS, GPI, NAAA, ALG1, CARM1, NGLY1, NME3, NT5E, P4HA2, TFRC, NDUFB11, FABP5, ATP5PB, SDHD, TXN, FAM3C, YBX1, CEACAM1, S100A13, GBP1, CDC23, SEMA3C, THEM6, ADAM9, NCEH1, P3H1, CASP7, DHX8, ERAP1, LNPEP, DNASE1, IDE, TEX264, LGALS9, GOLM1, HDGF, CKLF, MYDGF, ITFG1, HINT2, PF4, MRPL2, MRPL24, SERPINH1, HSPA1A, YBX1, CD9, ERLIN2, DAG1, ERLEC1, GNAS, MAGT1, OS9, PDZD8, EMC10, MIA2, POGLUT3, PRXL2A, RTF1, RCN3, BLTP2, RCN1, SDF2L1, SDF4, and variants thereof.

In certain embodiments, the rejuvenating factor is an enzyme selected from the group comprising of AUH, CMPK1, FKBP7, ST3GAL2, QPCT, USP11, ACE, CTSA, CTSH, GALC, GLA, HEXA, HMGCL, GUSB, LCAT, OXCT1, PCYOX1L, KDM1A, MYG1, DPP7, SLPI, DDX17, MSRB2, PTPRO, DNASE1L1, KDM3B, SCPEP1, ATPAF1, D2HGDH, SULF2, MINPP1, PLOD2, CES2, LIPA, PON2, ARSB, CES1, GLS, GPI, NAAA, ALG1, CARM1, NGLY1, NME3, NT5E, P4HA2, TFRC, THEM6, ADAM9, NCEH1, P3H1, CASP7, DHX8, ERAP1, LNPEP, DNASE1, IDE, and variants thereof.

In certain embodiments, the rejuvenating factor is an metabolic protein selected from the group comprising of AUH, CMPK1, FKBP7, ST3GAL2, QPCT, USP11, ACE, CTSA, CTSH, GALC, GLA, HEXA, HMGCL, GUSB, LCAT, OXCT1, MTMR14, MINPP1, PLOD2, CES2, LIPA, PON2, ARSB, CES1, GLS, GPI, NAAA, ALG1, CARM1, NGLY1, NME3, NT5E, P4HA2, TFRC, NDUFB11, FABP5, ATP5PB, SDHD, TXN, FAM3C, and variants thereof.

In certain embodiments, the rejuvenating factor is a CD marker selected from the group comprising PVR, CD55, CD40, CD163, CSF2RA, FAS, FCAR, ITGA4, PDCD1LG2, LILRB1, CD14, NRP1, ALCAM, NT5E, and variants thereof.

In certain embodiments, the rejuvenating factor is a skin rejuvenating factor.

As used herein, the term "hallmark(s) of aging" refers to biochemical changes that occur while aging and may lead to a progressive loss of physiological integrity, impaired function and, eventually, death. Hallmarks of aging manifests during chronological aging. They are defined as "(1) their age-associated manifestation, (2) the acceleration of aging by experimentally accentuating them, and (3) the opportunity to decelerate, stop, or reverse aging by therapeutic interventions on them" (Lopez-Otin, Blasco, Partridge, Serrano, & Kroemer, 2023). For example, hallmarks of aging are genome instability, telomere shortening or telomere attrition, epigenetic alterations, loss of proteostasis, macroautophagy, deregulated nutrient sensing, mitochondrial dysfunction, cellular senescence, altered intercellular communication, chronic inflammation and/or dysbiosis.

In certain embodiments, determining hallmarks of aging according to the method of the invention comprises analysing Ki67 positive cells, DNA methylation (epigenetic clocks), gene expression, mitochondrial membrane potential, reactive oxygen species (ROS), expression of β-Galactosidase, production of procollagen 1, production of hyaluronic acid, and/or production of GDF-11. In certain embodiments, the outcome of the comparison of said hallmarks of aging according to a method according to the invention indicates a rejuvenating effect of an active agent of the one or more rejuvenating factor(s) of the skin tissue cells. In certain embodiments, the outcome of the comparison of the expression level of one or more rejuvenating factor(s) of the skin tissue cells according to the method according to the invention indicates the ability of the active agent to induce or inhibit the production of one or more rejuvenating factor(s) of the skin tissue cells.

The amount of Ki67 positive cells as well as the production of collagen declines in aged skin, so that the amount of Ki67 positive cells and the production of procollagen 1 represent important aging biomarkers. Beta-galactosidase is expressed by senescent cells, which increase with age. The expression of ß-galactosidase is therefore also a hallmark of aging. The mitochondrial membrane potential, the amount of reactive oxygen species (ROS), DNA methylation patterns of the cultured cells and the potential of fibroblasts to differentiate to adipocytes, and production hyaluronic acid represent other hallmarks of aging.

Epigenetic clocks, which are based on DNA methylation values represent a molecular biomarker of aged human skin. In certain embodiments, analysing cultured cells is carried out by measuring DNA methylation, further analysed with the skin age clock (Falckenhayn, C., Bienkowska, A., Sohle, J., Wegner, K., Raddatz, G., Kristof, B., Gronniger, E. (2023). Identification of dihydromyricetin as a natural DNA methylation inhibitor with rejuvenating activity in human skin. Front Aging, 4, 1258184. d) and/or the blood age clock (Hannum, G., Guinney, J., Zhao, L., Zhang, L., Hughes, G., Sadda, S., Zhang, K. (2013). Genome-wide methylation profiles reveal quantitative views of human aging rates. Mol Cell, 49(2), 359-367.).

In certain embodiments, the result of a comparison of the expression level of one or more rejuvenating factor(s) of the skin tissue cells according to a method of the invention relates to the induction or inhibition of the production of a rejuvenating factor. In certain embodiments, the result of a comparison of the hallmarks of aging according to a method of the invention relates to the rejuvenating effect on the skin tissue cells.

In certain embodiments, the result of the comparison of the expression level according to a method of the invention relates to an induction or inhibition of the production of a rejuvenating factor. In certain embodiments, the induction or inhibition of the production of a rejuvenating factor is verified if the expression level is regulated in the same direction in at least 2 of 3 skin tissue cell samples, wherein the expression level is either upregulated or downregulated.

In certain embodiments, the result of the comparison of the hallmarks of aging according to a method of the invention relates to a rejuvenating effect on the skin tissue cells. In certain embodiments, the rejuvenating effect on the skin tissue cells is verified if the hallmarks of aging are regulated in the same direction in at least 2 of 3 skin tissue cell samples, wherein the hallmarks of aging are either upregulated or downregulated.

In certain embodiments, an active agent augments the effect of the one or more rejuvenating factor(s) identified by a method according to the invention. In certain embodiments, the active agent is an agonist or an antagonist. In certain embodiments, the active agent is an enzyme.

As used herein, the term "active agent" refers to any substance or agent that binds to a structure inside a skin tissue cell or on its surface and regulates the expression level and/or activity of at least one rejuvenating factor of the skin tissue cells identified by the method according to the invention.

In certain embodiments, provided is a composition comprising at least one active agent tested by the method according to the invention.

As used herein, the term "agonist" refers to any agonist that regulates the expression level and/or activity of at least one rejuvenating factor of the skin tissue cells identified by the method according to the invention. An agonist is a substance that binds to a structure inside a skin tissue cell or on its surface and causes a similar or the same action as the rejuvenating factor that normally binds to the structure. In certain embodiments, the agonist is mimicking the effect of the rejuvenating factor.

As used herein, the term "antagonist" refers to any antagonist that regulates the expression level/and or activity of at least one rejuvenating factor of the skin tissue cells identified by the method according to the invention. An antagonist is a substance that stops, i.e. inhibits or opposes, the action or effect of an agonist or of a rejuvenating factor. An antagonist binds to a structure inside a skin tissue cell or on its surface and makes it unable for the agonist or the rejuvenating factor to bind to the structure appropriately. As a result, the agonist(s) or the rejuvenating factor(s) are rendered ineffective.

As used herein, the term "enzyme" refers to any protein with catalytic properties, i.e. that acts as a catalyst in the skin tissue cells, regulating the rate at which chemical reactions proceed without itself being altered in the process.

The active agent may have a therapeutic effect and/or cosmetic effect on the human individual. A therapeutic effect is the treatment and/or prevention of a disease such as wrinkling, folds, sagging, age spots, uneven pigmentation, thinning, elasticity, scarring, surface roughness, surface vessels, redness, pore size, impaired wound healing, pale complexion, loss of elasticity and/or tightness. The active agent tested according to a method of the invention may have a prophylactic and/or curing effect on different states of aging of the skin, as well as a generally beneficial effect on the state of the skin. The active agents tested according to a method of the invention may be formulated for the treatment of wounds of different origins.

In certain embodiments, the use of the active agents tested according to a method of the invention may provide an alternative to the systemic administration, by replacing e.g. oral administration by topical administration.

A cosmetic effect is the improvement of appearance, such as treating and/or preventing the signs of phenotypical aging. Signs of phenotypical aging are, for example, wrinkling, folds, sagging, age spots, uneven pigmentation, thinning, elasticity, scarring, surface roughness, surface vessels, redness, pore size, impaired wound healing, pale complexion, loss of elasticity and/or tightness.

In certain embodiments, the active agent is a cosmetic agent. In certain embodiments, the agonist or antagonist is a cosmetic agent. A cosmetic agent when applied to an individual may promote attractiveness, alter appearance, beautify and/or cleanse. The cosmetic agent may also prevent the signs of phenotypical aging of human skin, which are for example wrinkling, folds, sagging, age spots, uneven pigmentation, thinning, elasticity, scarring, surface roughness, surface vessels, redness, pore size, impaired wound healing, pale complexion, loss of elasticity and/or tightness. In certain embodiments, the active agent tested according to a method of the invention may prevent the aging of the skin by combatting the formation of wrinkles.

In certain embodiments, the composition is a cosmetic composition. In certain embodiments, the cosmetic composition may be applied topically.

In certain embodiments, the present disclosure further provides the use of a cosmetic composition comprising at least one active agent tested by the method according to the invention. In certain embodiments, the cosmetic composition is applied topically. In certain embodiments, the present disclosure provides the use of a cosmetic comprising at least one active agent tested by the method according to the invention for moisturizing the skin.

In certain embodiments, the cosmetic composition is in the form of a lotion, creme, solution, gel, skin cleanser, oil, gels, hydrogel, powder, serum, foundation, facial mask, lip care product, sunscreen, exfoliants, ointments, and mixtures thereof.

In certain embodiments, an effective amount of the active agent comprised in the composition refers to about 10 ng/ml to about 100 ng/ml. For example, an effective amount of the active agent comprised in the composition refers to about 10 ng/ml, about 15 ng/ml, about 20 ng/ml, about 25 ng/ml. about 30 ng/ml, about 35 ng/ml, about 40 ng/ml, about 45 ng/ml, about 50 ng/ml, about 55 ng/ml, about 60 ng/ml, about 65 ng/ml, about 70 ng/ml, about 75 ng/ml, about 80 ng/ml, about 85 ng/ml, about 90 ng/ml, about 95 ng/ml or about 100 ng/ml.

The present disclosure further provides a rejuvenating factor for rejuvenating, augmenting, and/or repairing skin tissue of a human subject, identified by a method according to the invention. As used herein, "rejuvenating, augmenting, and/or repairing skin tissue of a human subject" may refer to the reduction of the signs of aging, such as wrinkling, folds, sagging, age spots, uneven pigmentation, thinning, elasticity, scarring, surface roughness, surface vessels, redness, pore size, impaired wound healing, pale complexion, loss of elasticity and/or tightness.

The present disclosure further provides a cosmetic composition for rejuvenating, augmenting, and/or repairing skin tissue of a human subject comprising at least one rejuvenating factor identified by a method according to the invention.

The present disclosure provides an agonist or antagonist, which regulates the expression of at least one rejuvenating factor identified according to a method of the invention.

The present disclosure further provides a method for rejuvenating, augmenting, and/or repairing skin tissue of a human subject, comprising the topical application of a composition according to the invention.

The present disclosure further provides a method for rejuvenating, augmenting, and/or repairing skin tissue of a human subject comprising the topical application of a cosmetic composition comprising at least one agonist or antagonist of at least one rejuvenating factor as identified by a method according to the invention.

It should be noted that the present invention may comprise any combination of the features and/or limitations referred to herein, except for combinations of such features which are mutually exclusive. The foregoing description is directed to particular embodiments of the present invention for the purpose of illustrating it. It will be apparent, however, to one skilled in the art, that many modifications and variations to the embodiments described herein are possible. All such modifications and variations are intended to be within the scope of the present invention, as defined in the appended claims.

The present disclosure may be described by one or more of the following paragraphs:

A. A method of culturing bone marrow cells in a culture device wherein a growth medium comprising age-specific human serum is added to the bone marrow cells.

B. The method according to paragraph A, wherein culturing bone marrow cells comprises co-culturing bone marrow cells with skin tissue cells.

C. The method according to paragraph A or B, wherein the bone marrow cells are human bone marrow cells.

D. The method according to any one of paragraphs A to C, wherein the culture device is a microfluidic culture device, preferably wherein the microfluidic culture device is a microfluidic organ-on-a-chip device.

E. The method according to any one of paragraphs A to D, wherein the age-specific human serum is young human serum from human donors aged 30 years or below.

F. The method according to any one of paragraphs A to D, wherein the age-specific human serum is old human serum from human donors aged 60 years or above.

G. The method according to any one of claims A to F, wherein the bone marrow cells are cultivated for a period of time of between about 1 to about 35 days.

H. A method of identifying one or more rejuvenating factor(s) comprising:

a) culturing bone marrow cells according to paragraph E,

b) analysing the protein expression of the cultured bone marrow cells of step a),

c) culturing bone marrow cells according to paragraph F,

d) analysing the protein expression of the cultured bone marrow cells of step c),

e) determining upregulated protein expression or downregulated protein expression by comparing the protein expression of a pair of bone marrow cell samples according to step b) and step d);

f) carrying out steps a) to e) for a total of 5 pairs of bone marrow cell samples;

g) selecting proteins that are regulated in the same direction in at least 4 out of 5 pairs of bone marrow cell samples; and

f) selecting proteins from step g) that belong to the human secretome.

I. The method according to paragraph H, wherein a protein is identified as rejuvenating factor if the protein is regulated in the same direction in at least 4 out of 5 bone marrow cell samples, wherein the protein is either upregulated or downregulated.

J. The method according to paragraph H or paragraph I, wherein the rejuvenating factor is an upregulated systemic protein with aging or a downregulated systemic protein with aging, such as proteins listed in Table 1.

K. The method according to any one of paragraphs claims H to J, wherein the upregulated systemic protein with aging is selected from the group comprising PLAU, SDCBP, SULF2, APOB, APOC1, APOC3, APOC2, MINPP, PLOD2, PON3, CES2, LIPA, PON2, FKBP10, PLA2G15, TGM2, ARSB, CES1, GLS, GPI, NAAA, ACP3, P4HA1, ALG1, CARM1, NGLY1, NME3, NT5E, P4HA2, PROZ, RDH11, TFRC, IL4I1, NDUFB11, FABP5, GLIPR1, ATP5PB, SDHD, TXN, FAM3C, AGT, YBX1, CEACAM1, ADGRE3, APOC4, APOF, APOM, APOE, S100A13, GBP1, PDCD1LG2, CDC23, COL22A1, COL6A1, COL6A2, COL6A3, CFHR1, C8G, C4A, SEMA3C, THEM6, ADAM9, NCEH1, P3H1, MMP7, PLBD2, CASP7, DHX8, ERAP1, LNPEP, PIK3AP1, DNASE1, IDE, FAP, TEX264, LGALS7, LGALS9, GOLM1, HDGF, CKLF, TGFBI, MYDGF, SHBG, IGKV6D-21, IGLV2-11, ITFG1, HINT2, PF4, MRPL2, MRPL24, SERPINH1, SERPINE1, HSPA1A, DNAJB11, YBX1, CD9, ERLIN2, CRYAB, DAG1, ERLEC1, GNAS, MAGT1, OS9, PDZD8, EMC10, MIA2, POGLUT3, CRP, SAA4, PRXL2A, RTF1, RCN3, BPIFB1, OOSP3, BLTP2, FCN1, HNRNPA2B1, RCN1, SDF2L1, SDF4, LAMC1, and variants thereof.

L. The method according to any one of claims H to J, wherein the downregulated systemic protein with aging is selected from the group comprising PVR, LILRB1, CD14, NRP1, ALCAM, IRAK3, SPINT1, ERO1A, CHIT1, LPL, AUH, CMPK1, FKBP7, ST3GAL2, QPCT, USP11, ACE, CTSA, CTSH, GALC, GLA, HEXA, HMGCL, PLA2G7, PTGS1, GUSB, LCAT, VNN1, OXCT1, CHI3L1, GPX3, ANGPTL4, SLC8A1, MTMR14, LAT2, IL1RN, S100A8, CD55, CD40, CD163, CSF2RA, FAS, FCAR, ITGA4, CCN1, EDEM2, EDEM3, EMILIN1, MFGE8, SEMG1, PCOLCE2, PCYOX1L, KDM1A, MYG1, DPP7, SLPI, CPA4, DDX17, MSRB2, PTPRO, MMP9, DNASE1L1, KDM3B, SCPEP1, ATPAF1, D2HGDH, FGG, CXCL5, IGHG1, IGHV2-5, IGHV3-15, IGHV3-20, IGHV3-35, IGHV4-28, IGKJ1, IGKV1-16, IGKV1-39, IGKV3-11, IGKV3D-15, IGKV6-21, IGLV3-21, IGLV4-69, IGLV7-46, IL18, PRG3, INS, PGLYRP2, SERPINB2, CNPY3, LBP, CETP, INTS3, MATR3, SAA1, MTX2, SYAP1, A2ML1, CST7, CIAO2A, PLBD1, SELENOF, SELENON, COLEC11, INHBA, SIGLEC7, HLA-C, ZG16B, CHID1, MESD, SIL1, TBL2, DMBT1, and variants thereof.

M. A method of testing an active agent to augment the effect of the one or more rejuvenating factor(s) identified by the method according to any one of paragraphs H to L, comprising the steps of:

a) providing skin tissue cells,

b) determining

i) the expression level of one or more rejuvenating factor(s) of the skin tissue cells identified by the method according to any one of paragraphs H to L, and/or

ii) the hallmarks of aging of the skin tissue cells,

c) contacting the skin cells of step a) with an active agent,

d) determining

i) the expression level of one or more rejuvenating factor(s) of the skin tissue cells identified by the method according to any one of paragraphs H to L, and/or

ii) the hallmarks of aging of the skin tissue cells,

e) comparing

    i) the expression level of one or more rejuvenating factor(s) determined in step b)i) with the expression level determined in step d)i), and/or

    ii) the hallmarks of aging determined in step b)ii) with the hallmarks of aging determined in step d)ii).

N. The method according to paragraph M, wherein the result of the comparison of the expression level according to step e)i) relates to the induction or inhibition of the production of an rejuvenating factor, and/or wherein the result of the comparison of the hallmarks of aging according to step e)ii) relates to the rejuvenating effect on the skin tissue cells.

O. The method according to paragraph N, wherein the induction or inhibition of the production of an rejuvenating factor is verified if the expression level according to step e)i) is regulated in the same direction in at least 2 of 3 skin tissue cell samples, wherein the expression level is either upregulated or downregulated; and/or wherein the rejuvenating effect on the skin tissue cells is verified if the hallmarks of aging according to step e)ii) are regulated in the same direction in at least 2 of 3 skin tissue cell samples, wherein the hallmarks of aging are either upregulated or downregulated.

P. The method according to any one of paragraphs M to O, wherein the active agent is an antagonist, an agonist or an enzyme.

Q. A composition comprising at least one active agent tested by the method according to any one of claims M to P.

R. The composition according to paragraph Q, wherein the composition is a cosmetic composition.

S. Use of a cosmetic composition according to paragraph R for rejuvenating, augmenting, and/or repairing skin tissue of a human subject.

T. The use of a cosmetic composition according to paragraph S, wherein the cosmetic composition is applied topically.

U. The composition according to paragraph T for use in a method for the treatment of wrinkling, folds, sagging, age spots, uneven pigmentation, thinning, elasticity, scarring, surface roughness, surface vessels, redness, pore size, impaired wound healing, pale complexion, loss of elasticity and/or tightness, wherein an effective amount of the composition is administered topically to the skin tissue of the human subject.

## EXAMPLES

Culturing bone marrow cells and skin tissue cells

### Pre-culturing bone marrow cells

500.000 precultured bone marrow cells hBM-MSCs (Lonza Group AG) were seeded onto a hydroxyapatite coated zirconiumoxide based Sponceram® cylinder (TissUse GmbH). After cultivation for 7 days in Gibco™ DMEM high glucose with 10 % FCS, 1 % P/S and 1 % GlutaMAX, 10.000 hBM CD34+ cells (Lonza Group AG) were added to the system and the medium changed to StemSpan™ SFEM II + 50 ng/mL SCF + 10 ng/mL TPO + 100 ng/mL Flt3-L + 1 % P/S.

### Culturing bone marrow cells

For dynamic cultivation, the cultivated Sponceram® cylinders were transferred into the 96-well compartment of the HUMIMIC Chip3plus. The circuit was filled with 2 ml fresh medium, and the system connected to the HUMIMIC Starter control unit. During the first two weeks, 1.6 ml of medium was exchanged twice a week. Once a week, the bone marrow cells were sampled through resuspension during the medium exchange for further flow cytometry analysis. After 14 days of dynamic bone marrow cell culture, the medium was changed to StemSpan™ SFEM II + 50 ng/mL SCF + 10 ng/mL TPO + 100 ng/mL Flt3-L + 1 % P/S. The co-culture was kept for further 21 days with a medium exchange twice a week of 1.6 ml. Additionally, bone marrow cells were collected once a week and analysed with flow cytometry.

### Co-culturing bone marrow cells and skin tissue cells

After 14 days of dynamic bone marrow cell culture, the skin models were added to the middle 24-well compartment of the HUMIMIC Chip3plus. Furthermore, the medium was changed to StemSpan™ SFEM II + 50 ng/mL SCF + 10 ng/mL TPO + 100 ng/mL Flt3-L + 1 % P/S + 10 % young human serum or old human serum. Age specific serum is added from day 14 on, i.e. at the start of the co-culture and remains at 10% serum throughout the whole co-culture. The co-culture was kept for further 21 days with a daily medium exchange of 1 ml. Additionally, bone marrow cells were collected once a week and analyzed with flow cytometry.

The skin models were extracted from the HUMIMIC Chip3plus after 3 weeks of co-culture and cut in half. While one half was directly frozen in Tissue-Tek® in liquid nitrogen for histology analysis, the other half was cut in half again. From those quarters, the epidermis and dermis were separated using tweezers and frozen separately in liquid nitrogen for further RNA and DNA analysis.

## Flow cytometry

From the HUMIMIC Chip3plus harvested bone marrow cells were transferred to a 96-well plate, filled with eBioscience flow cytometry staining buffer (Thermo Fisher Scientific) and centrifugated. All centrifugation steps were performed at 300 x g for 5 minutes at 4 °C. The supernatant was decanted, the cells resuspended in 25 $\mu$l FcR blocking reagent (1:12.5, Miltenyi Biotech) and incubated for 5 minutes on ice. Afterwards, 25 $\mu$l of the staining solution (1:50, BioLegend) was added, the cells were resuspended and incubated for further 20 minutes in the dark on ice. The wells were filled with 200 $\mu$l PBS (Gibco) and centrifugated again. After performing two washing steps with flow cytometry staining buffer and decanting the supernatant after every step, 50 $\mu$l of the viability staining solution (Zombie Green fixable viability kit, BioLegend) was added, incubated for 20 minutes in the dark on ice and washed again three times with 200 $\mu$l flow cytometry staining buffer. Finally, the cells were resuspended in 200 $\mu$l fresh flow cytometry staining buffer and measured at the flow cytometer (BD LSRFortessa™ Cell Analyzer, BD Biosciences). The cells were stained with combinations of the following antibodies: anti- CD123-PE, CD15-PE-Cy7, CD38-PerCP-Cy5.5, CD-45RA-BV421, CD16-APC, CD14-BV711, CD34-APC-Cy7, CD13-BV711, CD41-PE-Cy7, CD71-PerCP-Cy5.5, CD36-BV421, CD235a-APC, CD229-PE (BioLegend). The measured data was analyzed using FlowJo v10.7.1 software.

## Gene expression analysis

Frozen dermis and epidermis samples were thawed in 350 $\mu$l RLT lysis buffer (Qiagen) supplemented with 1 % $\beta$-mercaptoethanol and homogenized using ceramic beads and the Precellys 24 homogenizer (Bertin Technologies). The RNA was isolated according to protocol using the RNeasy Mini Kit (Qiagen) and the QiaCube Connect (Qiagen).
For nCounter XT (NanoString Technologies) gene expression analysis, 70 ng of RNA was used and hybridized using target-specific reporter and capture probes for 24 hours at 65 °C. The samples were measured using the nCounter SPRINT profiler system and analyzed with the nSolver software. Normalization was done to control samples and the following housekeeping genes: GAPDH, GUSB, OAZ1, PUM1 and UBC.

## DNA methylation analysis

Frozen skin tissue samples were thawed in PBS (-/-), homogenized using ceramic beads within the Precellys 24 homogenizer (Bertin Technologies) and the genomic DNA isolated according to protocol using the QIAamp DNA Investigator Kit (Qiagen). The quantity of DNA was determined using the Pico green Kit (Thermo Fisher Scientific) according to manufacturer instructions before analysis on the Methylation EPIC V2 microarray Chip (Illumina) measuring 935.000 CpG loci. Methylation data analysis was carried out using the R package minfi v1.48.0 (Aryee, M. J., Jaffe, A. E., Corrada-Bravo, H., Ladd-Acosta, C., Feinberg, A. P., Hansen, K. D., & Irizarry, R. A. (2014). Minfi: a flexible and comprehensive Bioconductor package for the analysis of Infinium DNA methylation microarrays. Bioinformatics, 30(10), 1363-1369).
Specifically, raw .idat files were loaded, filtered, pre-processed and quantile normalized by minfi with default parameters. Finally, quantile normalized data were used to determine the biological age of the skin tissue cells via skin DNA methylation clock (Falckenhayn, C., Bienkowska, A., Sohle, J., Wegner, K., Raddatz, G., Kristof, B., Gronniger, E. (2023). Identification of dihydromyricetin as a natural DNA methylation inhibitor with rejuvenating activity in human skin. Front Aging, 4, 1258184) and blood age clock (Hannum, G., Guinney, J., Zhao, L., Zhang, L., Hughes, G., Sadda, S., Zhang, K. (2013). Genome-wide methylation profiles reveal quantitative views of human aging rates. Mol Cell, 49(2), 359-367).

## Cytotoxicity analysis

During every medium exchange, the supernatant was collected. To measure the release of LDH, the Cytotoxicity Detection KitPLUS (LDH) (Roche) was used, and the supernatant measured directly. The remaining sample was frozen at -80 °C. To determine the percentage cytotoxicity, the maximum releasable LDH of the bone marrow cells and the skin tissue cells was quantified. The lysis buffer was added to control cells at the end of the experiment, incubated for 2 hours and the LDH release measured according to protocol. Finally, the cytotoxicity was calculated with the following equation:

Cytotoxicity [%] = (measured value – medium control) / (high control – medium control) x 100.

Gene Ontology analysis

The functional enrichment analysis was performed using g:Profiler (version e111_eg58_p18_f463989d) with g:SCS multiple testing correction method applying significance threshold of 0.05 (Kolberg et al., 2023). The either up- or downregulated proteins were introduced into the software and analysed. The highlighted driver terms (grouping significant terms into subontologies based on their relations and leading gene sets that give rise to other significant functions in the neighborhood) are depicted.

Mitochondrial membrane potential

The JC-10 Mitochondrial Membrane Potential (MMP) Assay Kit (Abcam) was used according to manufacturer's instructions to determine the MMP of bone marrow cells cultured with either young human serum or old human serum.

LC-MS/MS proteomics

The proteins produced by bone marrow cell models cultured with young or old human serum were analyzed. Cell lysates were prepared from 50,000 cells using an 8 M thiourea buffer, the protein concentration determined using the Invitrogen™ Qubit™ Protein Assay Kit and the subsequent protein digestion and peptide purification conducted in accordance with the filter-aided sample preparation protocol (Wiśniewski, 2018), including reduction, alkylation, and dual enzymatic digestion with rLys-C and trypsin. The desalted peptides were then evaporated and resuspended in 0.1 % formic acid in preparation for analysis. A total of 400 ng of peptides were separated via nano-uHPLC and analysed on a timsTOF Pro 2 mass spectrometer using DIA-PASEF, which was optimised with py_diAID (Skowronek et al., 2022). The raw data were processed with DIA-NN (version 1.8.1; (Demichev, Messner, Vernardis, Lilley, & Ralser, 2020) and a human reference proteome for library-free analysis. The assessment of significant differential abundant proteins were identified using DESeq2 (Love, Huber, & Anders, 2014), threshold adj. p-value <0.05)

Example 1 - Cultivation of bone marrow cells and skin tissue cells

Bone marrow cells including different myeloid immune cells and skin tissue cells were co-cultured within a HUMIMIC Chip3plus according to the methods provided in *Co-Culturing bone marrow cells and skin tissue cells* herein above.
Human bone marrow mesenchymal stem cells (MSCs) were seeded on a hydroxyapatite coated zirconiumoxide based Sponceram® and cultivated to create a human bone marrow niche like environment. Onto this scaffold, hBM CD34+ cells were added and differentiated towards myeloid cells within the HUMIMIC Chip3plus. After two weeks, the co-culture with the skin tissue cells (Phenion® full-thickness long life insert skin model) started and was kept for another 21 days.
A hematoxylin and eosin staining showed well-developed intact skin tissue cells including a dermis, epidermis, and stratum corneum over the entire cultivation time of 21 days (Figure 1 (c)).
The cultured cells were further analysed according to the method provided in *Cytotoxicity analysis* herein above. The cells were viable over the whole cultivation time as determined by a cytotoxicity below 5 % (Figure 1 (b)).
Three essential skin structure and differentiation markers were examined (collagen IV (Col IV), keratin 14 (Kr14) and keratin 10 (Kr10). While Col IV showed the well-developed basal membrane, Kr14 and Kr10 represent different stages of the keratinocyte differentiation. Both early (Kr14) and late (Kr10) keratinocyte differentiation were present in the co-cultured skin tissue cells during the whole course of culture (Figure 1 (c)).
Using *flow cytometry* as described herein above, the cell composition of bone marrow cells was analysed over the entire cultivation time of 35 days (Figure 1 (d)). After seven days of pre-culturing bone marrow cells, a quarter of the cell population were progenitor cells, while some monocytes and granulocytes, early erythroid cells as well as a small portion of megakaryocytes and platelets had already developed. Over time, the portion of progenitor cells decreased, while more monocytes and granulocytes developed. Furthermore, the percentage of early erythroid cells was diminished, and the number of megakaryocytes and platelets increased. Hence, all myeloid cells within the created bone marrow cells were present and all cell populations could be detected over the entire cultivation time. Investigating more closely the progenitor cells, the decline of HSCs, multipotent progenitors (MPPs) and common lymphoid progenitor cells (CLPs) was observed. On the other hand, common myeloid progenitor cells (CMPs), granulocyte-monocyte progenitor cells (GMPs) and megakaryocyte erythroid progenitor cells (MEPs) developed at a later stage and took the major share from culture day 21 on (Figure 1 (d), right).

Example 2 - Analysis of skin tissue cells after addition of young human serum or old human serum

Young human serum or old human serum has been added to the dynamic co-culture followed by analysing the co-cultured skin tissue cells to determine whether the skin tissue would be rejuvenated or aged.

A significant increase in Ki 67 positive cells in the dynamic skin model co-cultured with bone marrow cells and young serum compared to the model co-cultured with bone marrow cells and old serum was detected, indicating an improved regenerative capacity of the tissue (Figure 2 (b)).

*DNA methylation analysis* performed as described herein above predicted a significantly decreased biological age in skin tissue cells cultured with bone marrow cells and young serum compared to the culture with old serum. To further investigate the underlying epigenetic changes, the amount of 5-methyl-cytosines within the skin tissue cells was examined (Figure 2 (c)). A significant decrease in the amount of methylated cytosines after the dynamic co-culture with young human serum could be detected.

Example 3 - Analysis of bone marrow cells after addition of young human serum or old human serum

Young human serum or old human serum has been added to the dynamic co-culture followed by analysing the co-cultured bone marrow cells.

Cultivation with young serum resulted in significantly more progenitor cells on culture day 35 (Figure 3 (a), left). Beforehand, over the entire co-cultivation time, a clear trend of higher numbers of progenitor cells comparing the cultivation with young to old serum was detected. Moreover, among the further differentiated cells, the proportion of monocytes was decreased in bone marrow cells treated with young serum. In contrast, the portion of granulocytes was significantly increased after 35 days of the cultivation with young serum (Figure 3 (a), right). In summary, the significant increase of progenitor cells together with the significant decline of granulocytes after the cultivation with young serum, showed characteristics of a young bone marrow cells.

The *mitochondrial membrane potential* of the bone marrow cells was measured as described herein above as another hallmark of aging. The membrane potential was significantly higher in bone marrow cells cultured with young serum on day 24 and 31 (Figure 3 (b)). The increased amount of progenitor cells and the improved mitochondrial membrane potential together indicate that cultivation with young human serum was able to rejuvenate the bone marrow cells.

Furthermore, the biological age of the bone marrow cells was determined after 7 days of co-cultivation (i.e. after 21 days of cultivation including pre-cultivation) with either young human serum or old human serum. The age clock was significantly reduced after the cultivation with young serum in comparison to the control without serum (Figure 3 (c)). In summary, the significant increase of progenitor cells together with the significant decline of granulocytes, the improved mitochondrial membrane potential as well as the reduced biological age after the cultivation with young serum reproduce characteristics found in bone marrow cells of young compared to old individuals (Zeng, X., Shi, C., Han, Y., Hu, K., Li, X., Wei, C., Huang, H. (2024). A metabolic atlas of blood cells in young and aged mice identifies uridine as a metabolite to rejuvenate aged hematopoietic stem cells. Nat Aging).

Example 4 - Identification of rejuvenating factors

Bone marrow cells have been cultured as described in *Culturing bone marrow cells* herein above in addition of human age-specific serum followed by analysing the cultured cells to identify one or more rejuvenating factor(s).

Bone marrow cells have been analysed after 21 days of culture with either young human serum or old human serum or without age-specific serum on culture day 35 using LC-MS/MS *proteomics* as provided herein above. For analysis, bone marrow cells cultured with either young human serum or old human serum have been compared to bone marrow cells cultured without age-specific serum. Afterwards, bone marrow cells with young human serum have been compared to bone marrow cells cultured with old human serum.

Approximately 6.000 different proteins could be detected in all samples. 9 proteins were significantly differentially regulated between bone marrow treated with young and old human serum, among them 5 up- and 4 downregulated proteins (Figure 4 (a)) and 2078 were regulated in the same direction in at least 4 of 5 samples, with 1029 proteins downregulated and 1040 upregulated in bone marrow models cultured with old compared to young human serum (Figure 4 (a)). The regulated proteins have been compared to the 2772 known secretome proteins creating an overlap of 233 proteins. In more detail, of the 1033 downregulated proteins, 114 belonged to the secretome, while 125 of the 1045 upregulated proteins belonged to the secretome as well (Figure 4 (b)).

*Gene Ontology analysis* of the down- and upregulated proteins that also belonged to the human secretome was performed as described herein above (Figure 4 (c)). Different age-relevant regulated biological processes regarding cell death and apoptosis, as well as lipid metabolism and immune system processes were downregulated with aged serum. On the other hand, responses to stress and stimuli, as well as transport und triglyceride processes were upregulated after cultivation with old human serum.

Moreover, the present inventors determined the overlap of the regulated secretome proteins with proteins that have already been reported in multiple studies to significantly change upon aging inside the human plasma (Coenen, Lehallier, de Vries, & Middeldorp, 2023; Lehallier et al., 2019; Moaddel et al., 2021). The present inventors found an overlap of 55 proteins that were regulated in the system, potentially secreted and have been already described previously (Figure 4 (d)). Half of those proteins were down-, and the other half upregulated during the aging process. Looking at the protein classes of the overlapped proteins, all belonged to plasma proteins, and some CD markers, transporters, and enzymes could be detected. To further examine these proteins, a STRING network analysis (Szklarczyk, D., Kirsch, R., Koutrouli, M., Nastou, K., Mehryary, F., Hachilif, R., von Mering, C. (2023). The STRING database in 2023: protein-protein association networks and functional enrichment analyses for any sequenced genome of interest. Nucleic Acids Res, 51(D1), D638-D646) of the either down- or upregulated proteins was performed (Figure 4 (e)). The proteins expressed by either granulocytes or progenitor cells (both significantly reduced after the cultivation with old serum) were highlighted in the downregulated overlapped secretome proteins upon aged serum (Figure 4 (e), left). The upregulated overlapped secretome proteins expressed by monocytes (which increased with old serum) are highlighted as well (Figure 4 (e)). Both in down and upregulated proteins, the STRING network analysis showed some interconnected proteins. For example, the well-known metalloproteinase 9 (MMP9) was downregulated and showed interactions with 13 additional downregulated proteins. This indicates the importance of extracellular matrix regulation that might be crucial for skin homeostasis. Furthermore, MMP9 might be involved in HSCs recruitment and, as it was shown that the progenitor cell population decreases with old human serum, as well as upon aging in humans, the downregulation of this protein after treatment with old human serum completes the circle (Heissig. (2002). Recruitment of Stem and Progenitor Cells from the Bone Marrow Niche Requires MMP-9 Mediated Release of Kit-Ligand). Looking at the upregulated proteins, the heat shock protein 1 (HSPA1A) with its 15 connections and its induction in response to stressors relevant to aging is a well described and important regulator (Gomez, C. R. (2021). Role of heat shock proteins in aging and chronic inflammatory diseases. Geroscience, 43(5), 2515-2532).

Example 5 - Validation of rejuvenating factors

Since the age-associated proteins secreted by the bone marrow cells which were downregulated upon treatment with old human serum had the potential to be factors with rejuvenating effects on the skin, the present inventors tried to verify their ability to reverse different aging markers after supplementing either old fibroblasts or old keratinocytes with 100 ng/ml for 72 hours of each identified factor to be validated. As a benchmark control, the present inventors compared effects to GDF-11, a systemic factor with reported rejuvenating effects on human skin *in vitro* and ex *vivo.*

The analysis of Ki67 positive cells revealed a significant increase of proliferation of fibroblasts after treatment with Cystatin-F (CST7), Kunitz-type protease inhibitor 1 (SPINT1), Matrix metalloproteinase-9 (MMP-9), Fc fragment of IgA receptor (FCAR), Chitinase 3 like (CHI3L) and GDF-11 compared to the control without protein. In keratinocytes, treatment with the same proteins did not affect proliferation, but neither did GDF-11 (Figure 5 (a)).

Using the aging panel from before, between three to seventeen genes such as DPT, DCN, THBS1 and TAGLN were significantly differentially expressed after the addition of our newly identified proteins, except for FCAR (Figure 5 (b)).

Additionally, investigation of the supernatant of treated fibroblasts revealed significantly increased production of procollagen 1 after treatment with CST7, Complement decay-accelerating factor (CD55), SPINT1, MMP9, CHI3L and GDF-11 (Figure 5 (c)), whereas hyaluronic acid was significantly increased after addition of CD55, MMP9, CHI3L and GDF-11 (Figure 5 (d)).

To measure another integrative hallmark of aging, the ability to transdifferentiate into adipocyte-like cells was examined as a readout indicating partial reprogramming of fibroblasts to a phenotype with higher plasticity. After treatment with preadipocyte growth medium for two weeks, the culture with CD55 and CHI3L resulted in a significantly higher percentage of adipocyte-like cells compared to the control (Figure 5 (e)).

Consequently, the present inventors could show that five proteins were able to alleviate more than one hallmark of aging assay in human dermal fibroblasts, supporting their potential role in the observed bone marrow cell-derived cell mediated skin rejuvenation upon exposure to young serum.

These data underlie the importance of systemic interactions within the aging process. Human skin can be rejuvenated through the coaction of young systemic factors and proteins secreted by young bone marrow cells.

**Claims**

1. A method of culturing bone marrow cells in a culture device wherein a growth medium comprising age-specific human serum is added to the bone marrow cells.

2. The method according to claim 1, wherein culturing bone marrow cells comprises co-culturing bone marrow cells with skin tissue cells.

3. The method according to claim 1 or claim 2, wherein the bone marrow cells are human bone marrow cells.

4. The method according to any one of claims 1 to 3, wherein the culture device is a microfluidic culture device, preferably wherein the microfluidic culture device is a microfluidic organ-on-a-chip device.

5. The method according to any one of claims 1 to 4, wherein the age-specific human serum is young human serum from human donors aged 30 years or below.

6. The method according to any one of claims 1 to 4, wherein the age-specific human serum is old human serum from human donors aged 60 years or above.

7. A method of identifying one or more rejuvenating factor(s) comprising:

a) culturing bone marrow cells according to claim 5;
b) analysing the protein expression of the cultured bone marrow cell sample of step a);
c) culturing bone marrow cells according to claim 6;
d) analysing the protein expression of the cultured bone marrow cell sample of step c);
e) determining upregulated protein expression or downregulated protein expression by comparing the protein expression of a pair of bone marrow cell samples according to step b) and step d);
f) carrying out steps a) to e) for a total of 5 pairs of bone marrow cell samples;
g) selecting proteins that are regulated in the same direction in at least 4 out of 5 pairs of bone marrow cell samples; and
f) selecting proteins from step g) that belong to the human secretome.

8. The method according to claim 7, wherein the one or more rejuvenating factor(s) is an upregulated systemic protein with aging or a downregulated systemic protein with aging, such as the proteins listed in Table 1.

9. The method according to claim 7 or claim 8, wherein the upregulated systemic protein with aging is selected from the group comprising PLAU, SDCBP, SULF2, APOB, APOC1, APOC3, APOC2, MINPP, PLOD2, PON3, CES2, LIPA, PON2, FKBP10, PLA2G15, TGM2, ARSB, CES1, GLS, GPI, NAAA, ACP3, P4HA1, ALG1, CARM1, NGLY1, NME3, NT5E, P4HA2, PROZ, RDH11, TFRC, IL4I1, NDUFB11, FABP5, GLIPR1, ATP5PB, SDHD, TXN, FAM3C, AGT, YBX1, CEACAM1, ADGRE3, APOC4, APOF, APOM, APOE, S100A13, GBP1, PDCD1LG2, CDC23, COL22A1, COL6A1, COL6A2, COL6A3, CFHR1, C8G, C4A, SEMA3C, THEM6,ADAM9, NCEH1, P3H1, MMP7, PLBD2, CASP7, DHX8, ERAP1, LNPEP, PIK3AP1, DNASE1, IDE, FAP, TEX264, LGALS7, LGALS9, GOLM1, HDGF, CKLF, TGFBI, MYDGF, SHBG, IGKV6D-21, IGLV2-11, ITFG1, HINT2, PF4, MRPL2, MRPL24, SERPINH1, SERPINE1, HSPA1A, DNAJB11, YBX1, CD9, ERLIN2, CRYAB, DAG1, ERLEC1, GNAS, MAGT1, OS9, PDZD8, EMC10, MIA2, POGLUT3, CRP, SAA4, PRXL2A, RTF1, RCN3, BPIFB1, OOSP3, BLTP2, FCN1, HNRNPA2B1, RCN1, SDF2L1, SDF4, LAMC1, and variants thereof.

10. The method according to claim 7 or claim 8, wherein the downregulated systemic protein with aging is selected from the group comprising PVR, LILRB1, CD14, NRP1, ALCAM, IRAK3, SPINT1, ERO1A, CHIT1, LPL, AUH, CMPK1, FKBP7, ST3GAL2, QPCT, USP11, ACE, CTSA, CTSH, GALC, GLA, HEXA, HMGCL, PLA2G7, PTGS1, GUSB, LCAT, VNN1, OXCT1, CHI3L1, GPX3, ANGPTL4, SLC8A1, MTMR14, LAT2, IL1RN, S100A8, CD55, CD40, CD163, CSF2RA, FAS, FCAR, ITGA4, CCN1, EDEM2, EDEM3, EMILIN1, MFGE8, SEMG1, PCOLCE2, PCYOX1L, KDM1A, MYG1, DPP7, SLPI, CPA4, DDX17, MSRB2, PTPRO, MMP9, DNASE1L1, KDM3B, SCPEP1, ATPAF1, D2HGDH, FGG, CXCL5, IGHG1, IGHV2-5, IGHV3-15, IGHV3-20, IGHV3-35, IGHV4-28, IGKJ1, IGKV1-16, IGKV1-39, IGKV3-11, IGKV3D-15, IGKV6-21, IGLV3-21, IGLV4-69, IGLV7-46, IL18, PRG3, INS, PGLYRP2, SERPINB2, CNPY3, LBP, CETP, INTS3, MATR3, SAA1, MTX2, SYAP1, A2ML1, CST7, CIAO2A, PLBD1, SELENOF, SELENON, COLEC11, INHBA, SIGLEC7, HLA-C, ZG16B, CHID1, MESD, SIL1, TBL2, DMBT1, and variants thereof.

11. A method of testing an active agent to augment the effect of the one or more rejuvenating factor(s) identified by the method according to any one of claims 7 to 10, comprising the steps of:

a) providing skin tissue cells,
b) determining

i) the expression level of one or more rejuvenating factor(s) of the skin tissue cells identified by the method according to any one of claims 7 to 10, and/or

ii) the hallmarks of aging of the skin tissue cells,

c) contacting the skin cells of step a) with an active agent,

d) determining

i) the expression level of one or more rejuvenating factor(s) of the skin tissue cells identified by the method according to any one of claims 7 to 10, and/or

ii) the hallmarks of aging of the skin tissue cells,

e) comparing

i) the expression level of one or more rejuvenating factor(s) determined in step b)i) with the expression level determined in step d)i), and/or

ii) the hallmarks of aging determined in step b)ii) with the hallmarks of aging determined in step d)ii).

12. The method according to claim 11, wherein the result of the comparison of the expression level according to step e)i) relates to the induction or inhibition of the production of an rejuvenating factor, and/or wherein the result of the comparison of the hallmarks of aging according to step e)ii) relates to the rejuvenating effect on the skin tissue cells.

13. The method according to claim 11, wherein the induction or inhibition of the production of an rejuvenating factor is verified if the expression level according to step e)i) is regulated in the same direction in at least 2 of 3 skin tissue cell samples, wherein the expression level is either upregulated or downregulated; and/or wherein the rejuvenating effect on the skin tissue cells is verified if the hallmarks of aging according to step e)ii) are regulated in the same direction in at least 2 of 3 skin tissue cell samples, wherein the hallmarks of aging are either upregulated or downregulated.

14. A composition comprising at least one active agent tested by the method according to any one of claims 11 to 13.

15. The composition according to claim 14 for use in a method for the treatment of wrinkling, folds, sagging, age spots, uneven pigmentation, thinning, elasticity, scarring, surface roughness, surface vessels, redness, pore size, impaired wound healing, pale complexion, loss of elasticity and/or tightness, wherein an effective amount of the composition is administered topically to the skin tissue of the human subject.

Figure 1

(a)

d-7  d-1   d0     d14   d35

dynamic co-cultivation of skin model and BM model

(b)

(c)

Col IV / DAPI  Kr14 / DAPI  Kr10 / DAPI

(d)

Progenitor cells
Monocytes
Granulocytes
Megakaryocytes / Platelets
Early erythroid cells
other diff cells

HSCs / MPPs
CLPs
CMPs
GMPs
MEPs

Figure 2

Figure 3

(a)

- nonregulated
- significantly upregulated
- significantly downregulated
- upregulated (4/5)
- downregulated (4/5)

(b)

(c)

downregulated
GO-Terms
of secretome

upregulated
GO-Terms
of secretome

(d)

regulated secretome
proteins

significant aging
secretome proteins

(e)

downregulated overlapped
secretome proteins

upregulated overlapped
secretome proteins

Figure 5

Europäisches
Patentamt

European
Patent Office

Office européen
des brevets

## EUROPEAN SEARCH REPORT

**Application Number**

EP 24 22 1299

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 11 041 144 B2 (ISOPOGEN PTY LTD [AU]) 22 June 2021 (2021-06-22) | 1,3,4 | INV.<br>G01N33/50<br>C12N5/0775 |
| Y | * abstract;<br>page 3, left-column, 2nd para; section "discussion and conclusions" on pages 7-8 * | 2,5,6 | |
| X | WO 2006/022091 A1 (NAT INST OF ADVANCED IND SCIEN [JP]; OHGUSHI HAJIME [JP] ET AL.) 2 March 2006 (2006-03-02) | 1,3,4 | |
| Y | * abstract;<br>claim 1 * | 2,5,6 | |
| X | US 2017/313984 A1 (HONMOU OSAMU [JP] ET AL) 2 November 2017 (2017-11-02) | 1,3,4 | |
| Y | * the claims * | 2,5,6 | |
| Y | ALLEN SOPHIE L. ET AL: "The effect of young and old ex vivo human serum on cellular protein synthesis and growth in an in vitro model of aging",<br>AMERICAN JOURNAL OF PHYSIOLOGY-CELL PHYSIOLOGY,<br>vol. 321, no. 1, 1 July 2021 (2021-07-01), pages C26-C37, XP093279537,<br>ISSN: 0363-6143, DOI: 10.1152/ajpcell.00093.2021<br>* abstract;<br>page C28, right-column, last para - page C29, left-column, 2nd para * | 2,5,6 | **TECHNICAL FIELDS SEARCHED (IPC)**<br><br>G01N<br>C12N |

-/--

The present search report has been drawn up for all claims

| Place of search | Date of completion of the search | Examiner |
|---|---|---|
| Munich | 21 May 2025 | Lindberg, Pia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 3**

Europäisches
Patentamt

European
Patent Office

Office européen
des brevets

## EUROPEAN SEARCH REPORT

**Application Number**

EP 24 22 1299

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KIM SI-NA ET AL: "The Effects of Human Bone Marrow-Derived Mesenchymal Stem Cell Conditioned Media Produced with Fetal Bovine Serum or Human Platelet Lysate on Skin Rejuvenation Characteristics", INTERNATIONAL JOURNAL OF STEM CELLS, vol. 14, no. 1, 28 February 2021 (2021-02-28), pages 94-102, XP093005527, DOI: 10.15283/ijsc20070 * the whole document, especially the abstract; page 95, right-column - page 98, right-column; figures 2 and 4 * | 1-15 | |
| A | KIM YOON-JIN ET AL: "Conditioned media from human umbilical cord blood-derived mesenchymal stem cells stimulate rejuvenation function in human skin", BIOCHEMISTRY AND BIOPHYSICS REPORTS, vol. 16, 1 December 2018 (2018-12-01), pages 96-102, XP093005524, ISSN: 2405-5808, DOI: 10.1016/j.bbrep.2018.10.007 * abstract; figure 4; section 3.5 on pages 100-101 * | 1-15 | **TECHNICAL FIELDS SEARCHED (IPC)** |

-/--

The present search report has been drawn up for all claims

| Place of search | Date of completion of the search | Examiner |
|---|---|---|
| Munich | 21 May 2025 | Lindberg, Pia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 24 22 1299

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIU MENG-NAN ET AL: "Rejuvenation of young blood on aging organs: Effects, circulating factors, and mechanisms", HELIYON, vol. 10, no. 12, 1 June 2024 (2024-06-01), page e32652, XP093278470, GB ISSN: 2405-8440, DOI: 10.1016/j.heliyon.2024.e32652 * the whole document, especially the abstract; figure 1 * | 1-15 | |
| A | MURAGLIA A. ET AL: "A simple cell proliferation assay and the inflammatory protein content show significant differences in human plasmas from young and old subjects", FRONTIERS IN BIOENGINEERING AND BIOTECHNOLOGY, vol. 12, 9 September 2024 (2024-09-09), XP093278471, CH ISSN: 2296-4185, DOI: 10.3389/fbioe.2024.1408499 * abstract; page 3, left-column, 2nd para; section "discussion and conclusions" on pages 7-8 * | 1-15 | **TECHNICAL FIELDS SEARCHED (IPC)** |

The present search report has been drawn up for all claims

| Place of search | Date of completion of the search | Examiner |
|---|---|---|
| Munich | 21 May 2025 | Lindberg, Pia |

page 3 of 3

## ANNEX TO THE EUROPEAN SEARCH REPORT
## ON EUROPEAN PATENT APPLICATION NO.

EP 24 22 1299

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2025

| Patent document cited in search report | | Publication date | Patent family member(s) | | Publication date |
|---|---|---|---|---|---|
| US 11041144 | B2 | 22-06-2021 | AU | 2014344795 A1 | 10-03-2016 |
| | | | BR | 112016009753 A2 | 02-05-2018 |
| | | | CA | 2929333 A1 | 07-05-2015 |
| | | | CN | 105765059 A | 13-07-2016 |
| | | | EP | 3066193 A1 | 14-09-2016 |
| | | | ES | 2984009 T3 | 28-10-2024 |
| | | | JP | 6643999 B2 | 12-02-2020 |
| | | | JP | 6947430 B2 | 13-10-2021 |
| | | | JP | 2017501740 A | 19-01-2017 |
| | | | JP | 2020022486 A | 13-02-2020 |
| | | | KR | 20160070167 A | 17-06-2016 |
| | | | RU | 2016119489 A | 11-12-2017 |
| | | | SG | 11201603407V A | 30-05-2016 |
| | | | US | 2015307844 A1 | 29-10-2015 |
| | | | US | 2018112181 A1 | 26-04-2018 |
| | | | WO | 2015061839 A1 | 07-05-2015 |
| WO 2006022091 | A1 | 02-03-2006 | JP | 2006055106 A | 02-03-2006 |
| | | | WO | 2006022091 A1 | 02-03-2006 |
| US 2017313984 | A1 | 02-11-2017 | DK | 2602310 T3 | 24-02-2020 |
| | | | EP | 2602310 A1 | 12-06-2013 |
| | | | ES | 2778925 T3 | 12-08-2020 |
| | | | JP | 5185470 B2 | 17-04-2013 |
| | | | JP | WO2012018040 A1 | 03-10-2013 |
| | | | KR | 20130041992 A | 25-04-2013 |
| | | | PL | 2602310 T3 | 13-07-2020 |
| | | | PT | 2602310 T | 11-03-2020 |
| | | | SG | 187698 A1 | 28-03-2013 |
| | | | US | 2013130382 A1 | 23-05-2013 |
| | | | US | 2017313984 A1 | 02-11-2017 |
| | | | WO | 2012018040 A1 | 09-02-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **PALOVICS, R** ; **KELLER, A.** ; **SCHAUM, N.** ; **TAN, W.** ; **FEHLMANN, T.** ; **BORJA, M.** ; **WYSS-CORAY, T.** Molecular hallmarks of heterochronic parabiosis at single-cell resolution. *Nature*, 2022, vol. 603 (7900), 309-314
- **FALCKENHAYN, C** ; **BIENKOWSKA, A.** ; **SOHLE, J.** ; **WEGNER, K.** ; **RADDATZ, G.** ; **KRISTOF, B.** ; **GRONNIGER, E.** Identification of dihydromyricetin as a natural DNA methylation inhibitor with rejuvenating activity in human skin. *Front Aging*, 2023, vol. 4, 1258184
- **HANNUM, G., GUINNEY, J.** ; **ZHAO, L.** ; **ZHANG, L.** ; **HUGHES, G.** ; **SADDA, S.** ; **ZHANG, K.** Genome-wide methylation profiles reveal quantitative views of human aging rates.. *Mol Cell*, 2013, vol. 49 (2), 359-367
- **HANNUM, G.** ; **GUINNEY, J.** ; **ZHAO, L.** ; **ZHANG, L** ; **HUGHES, G.** ; **SADDA, S.** ; **ZHANG, K.** Genome-wide methylation profiles reveal quantitative views of human aging rates. *Mol Cell*, 2013, vol. 49 (2), 359-367
- **UHLÉN, M.** ; **KARLSSON, M. J.** ; **HOBER, A** ; **SVENSSON, A.-S.** ; **SCHEFFEL, J.** ; **KOTOL, D** ; **SIVERTSSON, Å.** The human secretome. *Science Signaling*, 2019, vol. 12 (609), 0274
- **COENEN, L.** ; **LEHALLIER, B.** ; **DE VRIES, H. E.** ; **MIDDELDORP, J.** Markers of aging: Unsupervised integrated analyses of the human plasma proteome. *Front Aging*, 2023, vol. 4, 1112109
- **LEHALLIER, B.** ; **GATE, D.** ; **SCHAUM, N.** ; **NANASI, T.** ; **LEE, S. E.** ; **YOUSEF, H** ; **WYSS-CORAY, T.** Undulating changes in human plasma proteome profiles across the lifespan. *Nat Med*, 2019, vol. 25 (12), 1843-1850
- **MOADDEL, R.** ; **UBAIDA-MOHIEN, C.** ; **TANAKA, T.** ; **LYASHKOV, A.** ; **BASISTY, N** ; **SCHILLING, B.** ; **FERRUCCI, L**. Proteomics in aging research: A roadmap to clinical, translational research. *Aging Cell*, 2021, vol. 20 (4), 13325
- **FALCKENHAYN, C** ; **BIENKOWSKA, A.** ; **SOHLE, J** ; **WEGNER, K.** ; **RADDATZ, G.** ; **KRISTOF, B.** ; **GRONNIGER, E.** Identification of dihydromyricetin as a natural DNA methylation inhibitor with rejuvenating activity in human skin. *Front Aging*, 2023, vol. 4, 1258184
- **HANNUM, G.** ; **GUINNEY, J.** ; **ZHAO, L** ; **ZHANG, L.** ; **HUGHES, G.** ; **SADDA, S.** ; **ZHANG, K.** Genome-wide methylation profiles reveal quantitative views of human aging rates. *Mol Cell*, 2013, vol. 49 (2), 359-367
- **ARYEE, M. J.** ; **JAFFE, A. E.** ; **CORRADA-BRAVO, H.** ; **LADD-ACOSTA, C.** ; **FEINBERG, A. P.** ; **HANSEN, K. D.** ; **IRIZARRY, R. A.** Minfi: a flexible and comprehensive Bioconductor package for the analysis of Infinium DNA methylation microarrays. *Bioinformatics*, 2014, vol. 30 (10), 1363-1369
- **FALCKENHAYN, C.** ; **BIENKOWSKA, A.** ; **SOHLE, J.** ; **WEGNER, K.** ; **RADDATZ, G.** ; **KRISTOF, B.** ; **GRONNIGER, E.** Identification of dihydromyricetin as a natural DNA methylation inhibitor with rejuvenating activity in human skin. *Front Aging*, 2023, vol. 4, 1258184
- **HANNUM, G.** ; **GUINNEY, J.** ; **ZHAO, L** ; **ZHANG, L.** ; **HUGHES, G.** ; **SADDA, S.** ; **ZHANG, K.** Genome-wide methylation profiles reveal quantitative views of human aging rates.. *Mol Cell*, 2013, vol. 49 (2), 359-367
- **ZENG, X.** ; **SHI, C.** ; **HAN, Y.** ; **HU, K** ; **LI, X.** ; **WEI, C.** ; **HUANG, H.** A metabolic atlas of blood cells in young and aged mice identifies uridine as a metabolite to rejuvenate aged hematopoietic stem cells. *Nat Aging*, 2024
- **SZKLARCZYK, D** ; **KIRSCH, R.** ; **KOUTROULI, M.** ; **NASTOU, K.** ; **MEHRYARY, F.** ; **HACHILIF, R.** ; **MERING, C.** The STRING database in 2023: protein-protein association networks and functional enrichment analyses for any sequenced genome of interest. *Nucleic Acids Res*, 2023, vol. 51 (1), 638-646
- **GOMEZ, C. R.** Role of heat shock proteins in aging and chronic inflammatory diseases. *Geroscience*, 2021, vol. 43 (5), 2515-2532